# EUROPÄISCHE PATENTSCHRIFT

(11) **EP 1 531 819 B1**
(45) Veröffentlichungstag und Bekanntmachung des Hinweises auf die Patenterteilung: **24.12.2008**
(21) Anmeldenummer: 03753349.4
(22) Anmeldetag: 05.08.2003
(51) Int. Cl.: A61K 31/506, A61K 31/4439, A61P 29/00

(54) **VERWENDUNG VON I kappa B-KINASE INHIBITOREN IN DER SCHMERZTHERAPIE**
USE OF I kappa B KINASE INHIBITORS FOR THE TREATMENT OF PAIN
UTILISATION D'INHIBITEURS DE L'I kappa B KINASE DANS LE TRAITEMENT DE LA DOULEUR

(30) Priorität: 17.08.2002 DE 10237723
(43) Veröffentlichungstag der Anmeldung: 25.05.2005
(73) Patentinhaber: Sanofi-Aventis Deutschland GmbH, 65929 Frankfurt am Main (DE)
(72) Erfinder: MICHAELIS, Martin, 60325 Frankfurt (DE); RITZELER, Olaf, 65812 Bad Soden (DE); JAEHNE, Gerhard, 65929 Frankfurt (DE); RUDOLPHI, Karl, 55116 Mainz (DE); GEISSLINGER, Gerd, 65812 Bad Soden (DE); SCHAIBLE, Hans-Georg, 07743 Jena (DE)
(86) Internationale Anmeldenummer: PCT/EP2003/008628
(87) Internationale Veröffentlichungsnummer: WO 2004/022057

(56) Entgegenhaltungen:
- WO-A-01/00610
- WO-A-01/30774
- YIN M-J ET AL: "THE ANTI-INFLAMMATORY AGENTS ASPIRIN AND SALICYLATE INHIBIT THE ACTIVITY OF IKAPPAB KINASE-BETA" NATURE, MACMILLAN JOURNALS LTD. LONDON, GB, Bd. 396, 5. November 1998 (1998-11-05), Seiten 77-80, XP002938591 ISSN: 0028-0836
- HOTTELET MARIA ET AL: "Development of IkappaB kinase inhibitors as anti-inflammatory therapeutics" INFLAMMATION RESEARCH, Bd. 49, Nr. Supplement 2, August 2000 (2000-08), Seite S91 XP009023847 10th National Conference of the Inflammation Research Association;Hot Springs, Virginia, USA; September 24-28, 2000 ISSN: 1023-3830

## Beschreibung

Die Erfindung betrifft die Verwendung von IκB-Kinase Inhibitoren zur Behandlung von Schmerzen.

In den Patentanmeldungen WO 01/00610, WO 01/30774 und WO 01/68648 werden Verbindungen beschrieben, die NFκB modulieren können.
NFκB ist ein heterodimerer Transkriptionsfaktor, der eine Vielzahl von Gene aktivieren kann, die unter anderen für proinflammatorische Cytokine wie IL-1, IL-2, TNFα oder IL-6 kodieren. NFκB liegt im Cytosol von Zellen komplexiert mit seinem natürlich vorkommenden Inhibitor IκB vor. Die Stimulation von Zellen, beispielsweise durch Cytokine, führt zur Phosphorylierung und anschließenden proteolytischen Abbau von IκB Dieser proteolytische Abbau führt zur Aktivierung von NFκB, das anschließend in den Kern der Zelle wandert und dort eine Vielzahl von proinflammatorischen Genen aktiviert.
In Erkrankungen wie rheumatoider Arthritis (bei der Entzündung), Osteoarthritis, Asthma, Herzinfarkt, Alzheimer Erkrankungen oder Atherosklerose ist NFκB über das normale Maß hinaus aktiviert. Die Hemmung von NFκB ist auch in der Krebstherapie von Nutzen, da sie dort zur Verstärkung der Cytostatika Therapie eingesetzt wird. Es konnte gezeigt werden, dass Arzneimittel wie Glucocorticoide, Salicylate oder Goldsalze, die in der Rheumatherapie eingesetzt werden, an verschiedenen Stellen in die NFκB aktivierende Signalkette inhibierend eingreifen oder direkt mit der Transkription der Gene interferieren.
Der erste Schritt in der genannten Signalkaskade ist der Abbau von IκB. Diese Phosphorylierung wird durch die spezifische IκB-Kinase reguliert.

In der Behandlung von akuten und chronischen Schmerzen werden Pharmaka einer Vielzahl verschiedener Substanzgruppen eingesetzt. Die Schmerztherapie ist trotzdem auch heute noch nicht befriedigend gelöst. Das liegt vor allem an der nicht ausreichenden Wirkstärke der auf dem Markt befindlichen Analgetika.

In dem Bestreben wirksame Verbindungen zur Behandlung von Schmerzen zu erhalten, wurde nun gefunden, das IκB-Kinase Inhibitoren dazu einsetzbar sind. Insbesondere konnte in den benutzten Modellen eine Wirkstärke demonstriert werden, die derjenigen klassischer nichtsteroidaler Antiphlogistika klar überlegen ist.

Die Erfindung betrifft daher die Verwendung von IκB-Kinase Inhibitoren zur Herstellung von Arzneimitteln zur Behandlung von Schmerzen.

Unter dem Begriff "Schmerzen" werden akute Schmerzen und chronische Schmerzen verstanden. Beispiele für chronische Schmerzen sind:
chronische muskuloskeletären Erkrankungen wie Rückenschmerzen,
Schmerzen bei Regelblutungen,
Schmerzen bei Osteoarthrose oder Rheumatoider Arthritis,
Schmerzen bei Darmentzündung,
Schmerzen bei Herzmuskelentzündung,
Schmerzen bei Multipler Sklerose,
Schmerzen bei Neuritis,
Schmerzen bei Karzinomen und Sarkomen,
Schmerzen bei AIDS,
Schmerzen bei Chemotherapie,
Amputationsschmerz,
Trigeminus-Neuralgie,
Kopfschmerzen wie Migränekopfschmerz, oder
Neuropathische Schmerzen wie postherpetische Zosterneuralgie.

Beispiele für akute Schmerzen sind:
Schmerzen nach Verletzungen,
Postoperativer Schmerzen,
Schmerzen bei akutem Gichtanfall oder
akute Schmerzen nach kieferchirurgischen Eingriffen.

IκB-Kinase Inhibitoren sind beispielsweise Indol- oder Benzimidazolderivate wie sie in den Patentanmeldungen WO 01/00610 und WO 01/30774 beschrieben werden.

Die Erfindung betrifft die erfindungsgemäße Verwendung von der Verbindung der Formel la 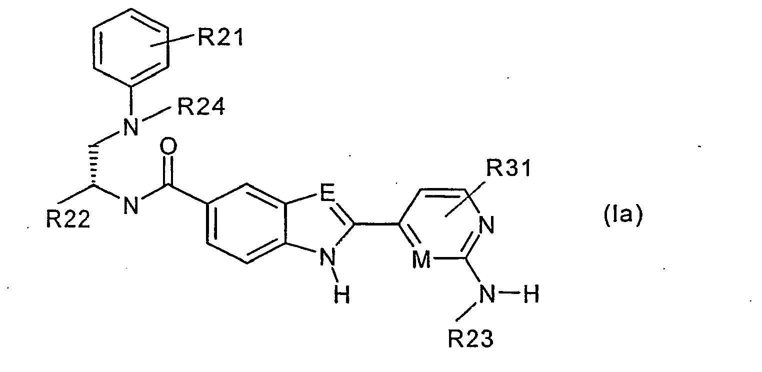 und/oder eine stereoisomere Form der Verbindung der Formel la und/oder ein physiologisch verträgliches Salz der Verbindung der Formel la, wobei
- E und M: gleich oder verschieden sind und unabhängig voneinander für N-Atom oder CH stehen,
- R21 und R31: gleich oder verschieden sind und unabhängig voneinander für
1. Wasserstoffatom,
2. Halogen,
3. -(C₁-C₄)-Alkyl,
4. -CN,
5. -CF₃,
6. -OR¹⁵, worin R¹⁵ für Wasserstoffatom oder -(C₁-C₄)-Alkyl steht,
7. -N(R¹⁵)-R¹⁶, worin R¹⁵ und R¹⁶ unabhängig voneinander für Wasserstoffatom oder -(C₁-C₄)-Alkyl stehen,
8. -C(O)-R¹⁵, worin R¹⁵ für Wasserstoffatom oder -(C₁-C₄)-Alkyl steht, oder
9. -S(O)_{X}-R¹⁵, worin x die ganze Zahl Null, 1 oder 2 bedeutet und R¹⁵ Wasserstoffatom oder -(C₁-C₄)-Alkyl bedeutet, stehen,
- R22: für
1. einen Heteroarylrest aus der Gruppe 3-Hydroxypyrro-2,4-dion, Imidazol, Imidazolidin, Imidazolin, Indazol, Isothiazol, Isothiazolidin, Isoxazol, 2-Isoxazolidin, Isoxazolidin, Isoxazolon, Morpholin, Oxazol, 1,3,4-Oxadiazol, Oxadiazolidindion, Oxadiazolon, 1,2,3,5-Oxathiadiazol-2-oxid, 5-Oxo-4,5-dihydro-[1,3,4]oxadiazol, 5-Oxo-1,2,4-thiadiazol, Piperazin, Pyrazin, Pyrazol, Pyrazolin, Pyrazolidin, Pyridazin, Pyrimidin, Tetrazol, Thiadiazol, Thiazol, Thiomorpholin, Triazol oder Triazolon, steht, und
   der Heteroarylrest unsubstituiert ist oder ein-, zwei- oder dreifach unabhängig voneinander substituiert ist durch
   1.1 -C(O)-R¹⁵, worin R¹⁵ für Wasserstoffatom oder -(C₁-C₄)-Alkyl steht,
   1.2 -(C₁-C₄)-Alkyl,
   1.3 -O-R¹⁵, worin R¹⁵ für Wasserstoffatom oder -(C₁-C₄)-Alkyl steht,
   1.7 -N(R¹⁵)-R¹⁶, worin R¹⁵ und R¹⁶ unabhängig voneinander für Wasserstoffatom oder -(C₁-C₄)-Alkyl stehen,
   1.8 Halogen oder
   1.9 Keto-Rest,
2. -C(O)-R¹⁵, worin R¹⁵ für Wasserstoffatom oder -(C₁-C₄)-Alkyl steht,
3. -C(O)-OR¹⁵, worin R¹⁵ für Wasserstoffatom oder -(C₁-C₄)-Alkyl steht, oder
4. -C(O)-N(R¹⁷)-R¹⁸, worin R¹⁷ und R¹⁸ unabhängig voneinander für Wasserstoffatom, -(C₁-C₄)-Alkyl-OH, -O-(C₁-C₄)-Alkyl oder -(C₁-C₄)-Alkyl stehen,
- R23 für: Wasserstoffatom oder -(C₁-C₄)-Alkyl steht,
- R24 für: 1. einen Heteroarylrest aus der Gruppe Pyrrol, Furan, Thiophen, Imidazol, Pyrazol,
Oxazol, Isoxazol, Thiazol, Isothiazol, Tetrazol, 1,2,3,5-Oxathiadiazol-2-Oxide, Triazolone, Oxadiazolon, Isoxazolon, Oxadiazolidindion, Triazol, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, β-Carbolin und benz-anellierte, cyclopenta-, oder cyclohexa- Derivate dieser Heteroarylreste,
   wobei der Heteroarylrest unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. einen Arylrest aus der Gruppe Phenyl, Naphthyl, 1-Naphthyl, 2-Naphthyl, Biphenylyl, 2-Biphenylyl, 3-Biphenylyl und 4-Biphenylyl, Anthryl oder Fluorenyl steht, und
   der Arylrest unsubstituiert ist oder ein-, zwei- oder dreifach unabhängig voneinander substituiert ist durch -(C₁-C₅)-Alkyl, -(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl.

Die Erfindung betrifft ferner die erfindungsgemäße Verwendung von der Verbindung der Formel la, wobei
- E und M: gleich oder verschieden sind und unabhängig voneinander für N-Atom oder CH stehen,
- R21 und R31: gleich oder verschieden sind und unabhängig voneinander für wie oben unter 1. bis 9. definiert sind,
- R22 für: 1. einen Heteroarylrest aus der Gruppe Imidazol, Isothiazol, Isoxazol, 2-Isoxazolidin, Isoxazolidin, Isoxazolon, 1,3,4-Oxadiazol, Oxadiazolidindion, 1,2,3,5-Oxadiazolon, Oxazol, 5-Oxo-4,5-dihydro-[1,3,4]oxadiazol, Tetrazol, Thiadiazol, Thiazol, Triazol oder Triazolon steht, und der Heteroarylrest unsubstituiert ist oder ein-, zwei- oder dreifach unabhängig voneinander substituiert ist durch
   1.1 Keto-Rest,
   1.2 Halogen oder
   1.3 -(C₁-C₂)-Alkyl, oder
2. -C(O)-N(R¹⁷)-R¹⁸, worin R¹⁷ und R¹⁸ unabhängig voneinander für Wasserstoffatom, -(C₁-C₄)-Alkyl-OH, -O-(C₁-C₄)-Alkyl oder -(C₁-C₄)-Alkyl stehen,
- R23 für: Wasserstoffatom, Methyl oder Ethyl steht,
- R24 für: 1. einen Heteroarylrest aus der Gruppe der ungesättigten, teilweise gesättigten oder vollständig gesättigte Ringe steht, die sich von Pyridin, Pyrazin, Pyrimidin, Pyridazin, Pyrrol, Furan, Thiophen, Imidazol, Pyrazol, Oxazol, Isoxazol, Thiazol, Triazol oder Isothiazol ableiten,
   wobei der Heteroarylrest unsubstituiert ist oder ein-, zwei- oder dreifach unabhängig voneinander substituiert ist durch -(C₁-C₄)-Alkyl, -(C₁-C₄)-Alkoxy, F, Cl, J, Br, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. Phenyl steht, und Phenyl unsubstituiert ist oder ein-, zwei- oder dreifach unabhängig voneinander substituiert ist durch F, Cl, J, Br, CF₃, -OH, -(C₁-C₄)-Alkyl oder -(C₁-C₄)-Alkoxy.

Die Erfindung betrifft ferner die erfindungsgemäße Verwendung der Verbindung 2-(2-Methylamino-pyrimidin-4-yl)-1H-indol-5-carbonsäure [(S)-2-diphenylamino-1-(5-oxo- 4,5-dihydro-[1,3,4]oxadiazol-2-yl)-ethyl]-amid oder 2-(2-Methylamino-pyrimidin-4-yl)-1H-benzoimidazol-5-carbonsäure ((S)1-carbamoyl-2- diphenylamin-ethyl)-amid.

Unter dem Begriff "Halogen" wird Fluor, Chlor, Brom oder Jod verstanden. Unter den Begriffen "-(C₁-C₈)-Alkyl", "-(C₁-C₆)-Alkyl" oder "-(C₁-C₄)-Alkyl" werden Kohlenwasserstoffreste verstanden, deren Kohlenstoffkette geradkettig oder verzweigt ist und 1 bis 8, 1 bis 6 bzw. 1 bis 4 Kohlenstoffatome enthält wie Methyl, Ethyl, Propyl, Butyl, Tertiär-Butyl, Pentyl, Hexyl, Heptyl oder Octyl. Unter dem Begriff "-(C₀)-Alkyl" wird eine kovalente Bindung verstanden. Cyclische Alkylreste sind beispielsweise 3- bis 6-gliedrige Monocyclen wie Cyclopropyl, Cyclobutyl, Cyclopentyl oder Cyclohexyl.

Unter dem Begriff "R⁸ und R⁹ bilden zusammen mit dem Stickstoff- und Kohlenstoffatom an das sie jeweils gebunden sind einen heterocyclischen Ring der Formel IIa" werden Reste verstanden die sich von Pyrrol, Pyrrolin, Pyrrolidin, Imidazol, Pyrazol, Oxazol, Isoxazol, Tetrazol, Isoxazolin, Isoxazolidin, Morpholin, Thiazol, Isothiazol, Isothiazolin, Purin, Isothiazolidin, Thiomorpholin, Pyridin, Piperidin, Pyrazin, Piperazin, Pyrimidin, Pyridazin, Indol, Isoindol, Indazol, Benzimidazol, Phthalazin, Chinolin, Isochinolin, Chinoxalin, Chinazolin, Cinnolin, Pteridin, Triazolone, Tetrazol, 1,2,3,5-Oxathiadiazol-2-Oxide, Oxadiazolon, Isoxazolon, Oxadiazolidindion, Triazol, welche durch F, -CN, -CF₃ oder -C(O)-O-(C₁-C₄)-Alkyl substituiert sind, 3-Hydroxypyrro-2,4-dione, 5-Oxo-1,2,4-Thiadiazole, Imidazolidin, -Carbolin und benz-anellierte Derivate dieser Heterocyclen ableiten.

Unter dem Begriff "R⁹ und Z bilden zusammen mit den Kohlenstoffatomen an das sie jeweils gebunden sind einen heterocyclischen Ring der Formel IIc" werden Reste verstanden, die sich von der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Tetrahydrochinolin, Tetrahydroisochinolin, Isochinolin, Tetrazol, 1,2,3,5-Oxathiadiazol-2-Oxide, Oxadiazolon, Isoxazolon, Triazolon, 3-Hydroxypyrro-2,4-dione, 1,3,4-Oxadiazol und 5-Oxo-1,2,4-Thiadiazol, Oxadiazolidindion, Triazol, welche unsubstituiert oder durch F, CN, CF₃ oder C(O)-O-(C₁-C₄)-Alkyl substituiert sind.

Unter dem Begriff "Heteroarylrest aus der Gruppe der ungesättigten, teilweise gesättigten oder vollständig gesättigten Ringe, die sich von Pyridin, Pyrazin, Pyrimidin, Pyridazin, Pyrrol, Furan, Thiophen, Imidazol, Pyrazol, Oxazol, Isoxazol, Thiazol, Isothiazol ableiten" werden beispielsweise Verbindungen verstanden wie Piperazin, Pyrazolin, Imidazolin, Pyrazolidin, Imidazolidin, Tetrahydropyridin, Isoxazolin, Isoxazolidin, Morpholin, Isothiazolin, Isothiazolidin, Tetrahydro-1,4-thiazin oder Piperidin.
Unter dem Begriff Aryl werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Arylreste, insbesondere Phenylreste, können einfach oder mehrfach, bevorzugt einfach, zweifach oder dreifach, durch gleiche oder verschiedene Reste substituiert sein, bevorzugt durch Reste aus der Reihe -(C₁-C₈)-Alkyl, insbesondere -(C₁-C₄)-Alkyl, -(C₁-C₈)-Alkoxy, insbesondere -(C₁-C₄)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy, Tetrazolyl substituiert sein. Entsprechendes gilt beispielsweise für Reste wie Arylalkyl oder Arylcarbonyl. Arylalkylreste sind insbesondere Benzyl sowie 1- und 2-Naphthylmethyl, 2-, 3- und 4-Biphenylylmethyl und 9-Fluorenylmethyl. Substituierte Arylalkylreste sind beispielsweise durch einen oder mehrere - (C₁-C₈)-Alkylreste, insbesondere -(C₁-C₄)-Alkylreste, im Arylteil substituierte Benzylreste und Naphthylmethylreste, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethyl-benzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere -(C₁-C₈)-Alkoxyreste, insbesondere -(C₁-C₄)-Alkoxyreste, im Arylteil substituierte Benzylreste und Naphthylmethylreste, zum Beispiel 4-Methoxybenzyl, 4-Neopentyloxybenzyl, 3,5-Dimethoxybenzyl, 3,4-Methylen-dioxybenzyl, 2,3,4-Trimethoxybenzyl, Nitrobenzylreste, zum Beispiel 2-, 3- und 4-Nitrobenzyl, Halobenzylreste, zum Beispiel 2-, 3- und 4-Chlor- und 2-, 3-, und 4-Fluorbenzyl, 3,4-Dichlorbenzyl, Pentafluorbenzyl, Trifluormethylbenzyl-Reste, zum Beispiel 3- und 4-Trifluormethylbenzyl oder 3,5-Bis(trifluormethyl)benzyl.

In monosubstituierten Phenylresten kann sich der Substituent in der 2-Position, der 3-Position oder der 4-Position befinden. Zweifach substituiertes Phenyl kann in der 2,3-Position, der 2,4-Position, der 2,5-Position, der 2,6-Position, der 3,4-Position oder der 3,5-Position substituiert sein. In dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Die Erläuterungen zu den Arylresten gelten entsprechend für zweiwertige Arylenreste, zum Beispiel für Phenylenreste, die beispielsweise als 1,4-Phenylen oder als 1,3-Phenylen vorliegen können. Phenylen-(C₁-C₆)-alkyl ist insbesondere Phenylenmethyl (-C₆H₄-CH₂-) und Phenylenethyl, (C₁-C₆)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Der Begriff "Heteroaryl mit 5 bis 14 Ringgliedern" steht für einen Rest eines monocyclischen oder polycyclischen aromatischen Systems mit 5 bis 14 Ringgliedern, das 1, 2, 3, 4 oder 5 Heteroatome als Ringglieder enthält. Beispiele für Heteroatome sind N, O und S. Sind mehrere Heteroatome enthalten, können diese gleich oder verschieden sein. Heteroarylreste können ebenfalls einfach oder mehrfach, bevorzugt einfach, zweifach oder dreifach, durch gleiche oder verschiedene Reste aus der Reihe ---(C₁-C₈)-Alkyl, insbesondere -(C₁-C₄)-Alkyl, -(C₁-C₈)-Alkoxy, insbesondere -(C₁-C₄)-Alkoxy, Halogen, Nitro, -N(R¹⁰)₂, Trifluormethyl, Hydroxy, Hydroxy-(C₁-C₄)-alkyl wie Hydroxymethyl oder 1-Hydroxyethyl oder 2-Hydroxyethyl, Methylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy, Tetrazolyl substituiert sein. Bevorzugt steht Heteroaryl mit 5 bis 14 Ringgliedern für einen monocyclischen oder bicyclischen aromatischen Rest, der 1, 2, 3 oder 4, insbesondere 1, 2 oder 3, gleiche oder verschiedene Heteroatome aus der Reihe N, Ö und S enthält und der durch 1, 2, 3 oder 4, insbesondere 1 bis 3, gleiche oder verschiedene Substituenten aus der Reihe -(C₁-C₆)-Alkyl, -(C₁-C₆)-Alkoxy, Fluor, Chlor, Nitro, -N(R¹⁰)₂, Trifluormethyl, Hydroxy, Hydroxy-(C₁-C₄)-alkyl, -(C₁-C₄)-Alkoxycarbonyl, Phenyl, Phenoxy, Benzyloxy und Benzyl substituiert sein kann. Besonders bevorzugt steht Heteroaryl für einen monocyclischen oder bicyclischen aromatischen Rest mit 5 bis 10 Ringgliedern, insbesondere für einen 5-gliedrigen bis 6-gliedrigen monocyclischen aromatischen Rest, der 1, 2 oder 3, insbesondere 1 oder 2, gleiche oder verschiedene Heteroatome aus der Reihe N, 0 und S enthält und durch 1 oder 2 gleiche oder verschiedene Substituenten aus der Reihe -(C₁-C₄)-Alkyl, Halogen, Hydroxy, -N(R¹⁰)₂, -(C₁-C₄)-Alkoxy, Phenyl, Phenoxy, Benzyloxy und Benzyl substituiert sein kann.

Der Begriff "Heterocyclus mit 5 bis 12 Ringgliedern" steht für einen monocyclischen oder bicyclischen 5-gliedrigen bis 12-gliedrigen heterocyclischen Ring, der teilweise gesättigt oder vollständig gesättigt ist. Beispiele für Heteroatome sind N, 0 und S. Der Herterocyclus ist unsubstituiert oder an einem oder mehreren Kohlenstoffatomen oder an einem oder mehreren Heteroatomen durch gleiche oder verschiedene Substituenten substituiert. Diese Substituenten sind oben beim Rest Heteroaryl definiert worden. Insbesondere ist der heterocyclische Ring einfach oder mehrfach, zum Beispiel einfach, zweifach, dreifach oder vierfach, an Kohlenstoffatomen durch gleiche oder verschiedene Reste aus der Reihe -(C₁-C₈)-Alkyl, zum Beispiel -(C₁-C₄)-Alkyl, -(C₁-C₈)-Alkoxy, zum Beispiel -(C₁-C₄)-Alkoxy wie Methoxy, Phenyl-(C₁-C₄)-alkoxy, zum Beispiel Benzyloxy, Hydroxy, Oxo, Halogen, Nitro, Amino oder Trifluormethyl substituiert und/oder er ist an den Ring-Stickstoffatome/en im heterocyclischen Ring durch -(C₁-C₈)-Alkyl, zum Beispiel -(C₁-C₄)-Alkyl wie Methyl oder Ethyl, durch gegebenenfalls substituiertes Phenyl oder Phenyl-(C₁-C₄)-alkyl, zum Beispiel Benzyl, substituiert. Stickstoffheterocyclen können auch als N-Oxide vorliegen oder als Quartärsalze. Beispiele für die Begriffe Heteroaryl mit 5 bis 14 Ringgliedern oder Heterocyclus mit 5 bis 12 Ringgliedern sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolon, Oxadiazolidindion, Triazol, welche durch F, -CN, -CF₃ oder-C(O)-O-(C₁-C₄)-Alkyl substituiert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Die allgemeine Strukturformel von α-Aminosäuren ist wie folgt:

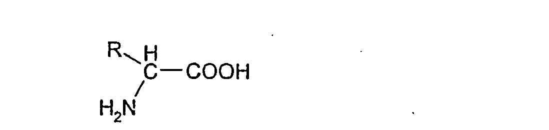

Die α-Aminosäuren unterscheiden sich untereinander durch den Rest R, der im Rahmen der vorliegenden Anmeldung als "charakteristischer Rest " einer Aminosäure bezeichnet wird. Für den Fall, dass R⁹ den charakteristischen Rest einer Aminosäure bedeutet, werden vorzugsweise die charakteristischen Reste der folgenden natürlich vorkommenden α-Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure eingesetzt. Insbesondere bevorzugt sind Histidin, Tryptophan, Serin, Threonin, Cystein, Methionin, Asparagin, Glutamin, Lysin, Arginin, Glutaminsäure und Asparaginsäure. Ferner sind bevorzugte charakteristische Reste einer Aminosäure die eingesetzt werden als Rest R⁹ auch nicht natürlich vorkommenden Aminosäuren wie 2-Aminoadipinsäure, 2-Aminobuttersäure, 2-Aminoisobuttersäure , 2,3-Diamino-propionsäure, 2,4-Diaminobuttersäure,1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Amino-heptansäure, Pipecolinsäure, Hydroxylysin, Sarkosin, N-Methylisoleucin, 6-N-Methyl-lysin, N-Methylvalin, Norvalin, Norleucin, Ornithin, allo-Isoleucin, allo-Threonin, allo-Hydroxylysin, 4-Hydroxyprolin, 3-Hydroxyprolin, 3-(2-Naphtyl)-alanin, 3-(1-Naphtyl-alanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, 2-Amino-3-phenylaminopropionsäure, 2-Amino-3-phenylaminoethylpropionsäure, Phosphinothricin, 4-Fluorphenylalanin, 3-Fluorphenylalanin, 4-Fluorphenylalanin, 3-Fluorphenylalanin, 3-Fluorphenylalanin, 2-Fluor-phenylalanin, 4-Chlorphenylalanin, 4-Nitrophenylalanin, 4-Aminophenylalanin, Cyclohexylalanin, Citrullin, 5-Fluortryptophan, 5-Methoxytryptophan, Methionin-Sulfon, Methionin-Sulfoxid oder -NH-NR¹¹-C(O)N(R¹¹)₂, die gegebenenfalls auch substituiert sind. Bei natürlichen aber auch nicht natürlichen vorkommenden Aminosäuren, die eine funktionelle Gruppe wie Amino, Hydroxy, Carboxy, Mercapto, Guanidyl, Imidazolyl oder Indolyl haben, kann diese Gruppe auch geschützt sein.

Als geeignete Schutzgruppe werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Aloc) oder vom Säureamid-Typ insbesondere Formyl, Acetyl oder Trifluoracetyl sowie vom Alkyl-Typ beispielsweise Benzyl. Für den Fall eines Imidazols-Restes in R⁸ dient beispielsweise das für die Sulfonamidbildung eingesetzte Sulfonsäurederivat der Formel IV als Schutzgruppe des Imidazol-Stickstoffs, die sich insbesondere in Gegenwart von Basen wie Natronlauge wieder abspalten lässt.

Die Herstellung der Verbindungen der Formeln I und Ia erfolgt wie beschrieben in den Patentanmeldungen WO 01/00610 und WO 01/30774. Die Ausgangsstoffe der chemischen Umsetzungen sind bekannt oder lassen sich nach Literatur bekannten Methoden leicht herstellen.

Aufgrund der pharmakologischen Eigenschaften der erfindungsgemäß eingesetzten IkB-Kinasen Inhibitoren, die sich in den benutzten Modellen zeigt, eignen sich die genannten Inhibitoren zum Einsatz in allen Schmerzformen, insbesondere bei Schmerzen, bei denen entzündliche Prozesse eine Rolle spielen.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Die orale oder intraartikuläre Applikation ist bevorzugt.

Die Erfindung betrifft auch ein Verfahren zur Herstellung eines Arzneimittels, das dadurch gekennzeichnet, dass man mindestens eine Verbindung der Formeln I oder la mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt. Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthalt. Bei festen Dosierungseinheiten wie Tabletten, Kapseln, Dragees oder Suppositorien, kann diese Dosis bis zu etwa 1000 mg, bevorzugt von etwa 50 mg bis 300 mg und bei Injektionslösungen in Ampullenform bis zu etwa 300 mg, vorzugsweise von etwa 10 mg bis 100 mg, betragen. Für die Behandlung eines erwachsenen, etwa 70 kg schweren Patienten sind je nach Wirksamkeit der Verbindung gemäß der Formeln I oder la, Tagesdosen von etwa 20 mg bis 1000 mg Wirkstoff, bevorzugt von etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt. Temperaturangaben in Grad Celsius, RT bedeutet Raumtemperatur (22 °C bis 26 °C). Verwendete Abkürzungen sind entweder erläutert oder entsprechen den üblichen Konventionen.
Nachfolgend ist die Erfindung an Hand von Beispielen näher erläutert.

### Herstellungsbeispiele

### A.1.) Synthese der Aminosäure ((S)-2-Amino-3-diphenylamino-propionsäure-methyl-ester (5))

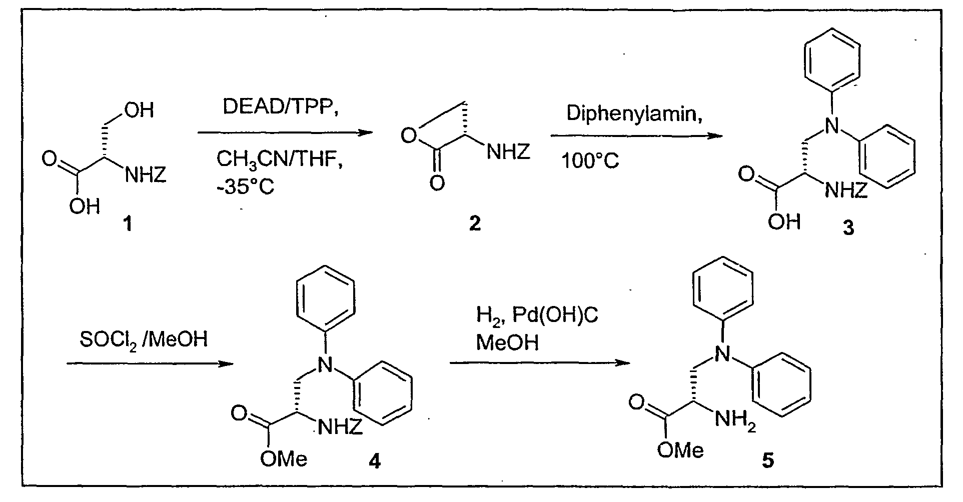

### N-Benzyloxy-carbonyl-L-serin-β-lacton (2)

54,8 g (0,209 mol) Triphenylphosphin wurden in 600 ml Acetonitril suspendiert und unter Ausschluss von Feuchtigkeit auf -35 °C bis -45 °C gekühlt. Bei dieser Temperatur wurde innerhalb von 50 Minuten tropfenweise 36,4 g (0,209 mol) Azodicarbon-säure-diethylester hinzugegeben. Man rührte 15 Minuten bei -35 °C nach. Zu diesem Gemisch tropfte man dann langsam eine Lösung aus 50 g (0,209 mol) N-Benzyloxycarbonyl-L-serin (**1**) in 500 ml Acetonitril, so dass die Temperatur nicht über -35 °C stieg. Anschließend wurden 12 h bei 5 °C gerührt. Zum Abbruch der Reaktion wurde die Reaktionslösung unter vermindertem Druck vom Lösungsmittel befreit und das Rohprodukt mit Mitteldruckchromatographie an Kieselgel gereinigt. (DCM/AcCN : 25/1) Nach dem Entfernen des Lösungsmittels erhielt man 20,8 g N-Benzyloxy-carbonyl-L-serin-β-lacton (2); Ausbeute 45 %; (siehe auch Org. Synth. 1991 (70) 1ff.) in feinen Nadeln.
Summenformel C₁₁H₁₁NO₄; M.W. = 221,2; MS (M+H) 222,1; ¹H NMR (DMSO-d₆) 4.30 (m, 1H), 4.45 (m, 1 H), 5.10 (s, 2H), 5.22 (m, 2H), 7.45 (m, 5H), 8.20 (d, J = 9.8 Hz, 1 H).

### (S)-2-Benzyloxycarbonylamino-3-diphenylamino-propionsäure (3)

5,0 g (22.6 mmol) Serinlacton (2) wurden mit 20g (118,2mmol) Diphenylamin verrührt und 2h auf 100°C erhitzt. Das Rohprodukt wurde durch Mitteldruckchromatographie an Kieselgel gereinigt. (DCM/Methanol : 9/1, anschließend EE/n-Heptan : 4/1) Nach Entfernen des Lösungsmittels erhielt man 3.65 g (Ausbeute 42%) saubere 2-Benzyloxycarbonylamino-3-diphenylamino-propionsäure (**3**).
Summenformel C₂₃H₂₂N₂O₄; M.W. = 390,44; MS (M+H) 391.2;
¹H NMR (DMSO-d₆) 3.85 (m, 1 H), 4.18 (m, 1 H), 4.3 (m, 1H), 4.9 (m, 2H), 6.9 (m, 5H), 7.25 (m, 10H).

### (S)-2-Benzyloxycarbonylamino-3-diphenylamino-propionsäure-methyl-ester (4)

Zu 75 ml Methanol wurden bei -5°C 6,5 ml (89,1 mmol) Thionylchlorid getropft und 15 min gerührt. Anschließend wurde 3,6 g (9.22 mmol) 2-Benzyloxycarbonylamino-3-diphenylamino-propionsäure (**3**) in 75 ml Methanol gelöst zugegeben und weitere 3 Stunden (h) bei Raumtemperatur gerührt. Nach der Evaporierung der Lösungsmittel wurde der Rückstand in Essigester aufgenommen und mit Natriumcarbonat Lösung extrahiert. Die Reinigung mittels Flash-Chromatographie (n-Heptan/Essigester 7:3) lieferte 2.76 g (50% Ausbeute) von 2-Benzyloxycarbonylamino-3-diphenylamino-propionsäure-methyl-ester (**4**). Summenformel C₂₄H₂₄N₂O₄; M.W. = 404,47; MS (M+H) 405.2;
¹H NMR (DMSO-d₆) 3.58 (s, 3H), 3.95 (m, 1H), 4.18 (m, 1H), 4.4 (m, 1H), 4.95 (m, 2H), 6.9 (m, 6H), 7.3 (m, 9H), 7.85 (d, J = 9.8 Hz, 1 H).

### (S)-2-Amino-3-diphenylamino-propionsäure-methyl-ester (5)

Zur Abspaltung der Z-Schutzgruppe löste man 2,7 g (6,68 mmol) des Z-geschützte Derivates (**4**) in 500 ml Methanol und unter Stickstoff Schutzatmosphäre wurden 100 mg Katalysator (10% Pd(OH)₂-C) zugeführt. Anschließend wurde das Inertgas mit einen großen Überschuss Wasserstoff verdrängt und 2 h in der Wasserstoffatmosphäre geschüttelt. Zum Abbruch der Reaktion wurde der Katalysator abfiltriert und das Filtrat eingeengt. Man erhielt 1.65 g (Ausbeute: 91%) 2-Amino-3-diphenylamino-propionsäure-methyl-ester (**5**).
Summenformel C₁₆H₁₈N₂O₂; M.W. = 270,32; MS (M+H) 271.2;
¹H NMR (DMSO-d₆) 3.45 (s, 3H), 3.58 (m, 1H), 3.8 (m, 1H), 3.95 (m, 1H), 6.9 (m, 6H), 7.3 (m, 4H).

### A.2.) Synthese des heterozyklischen Grundkörpers (2-(2-Methylamino-pyrimidin-4-yl)-1H-indol-5-carbonsäure (10))

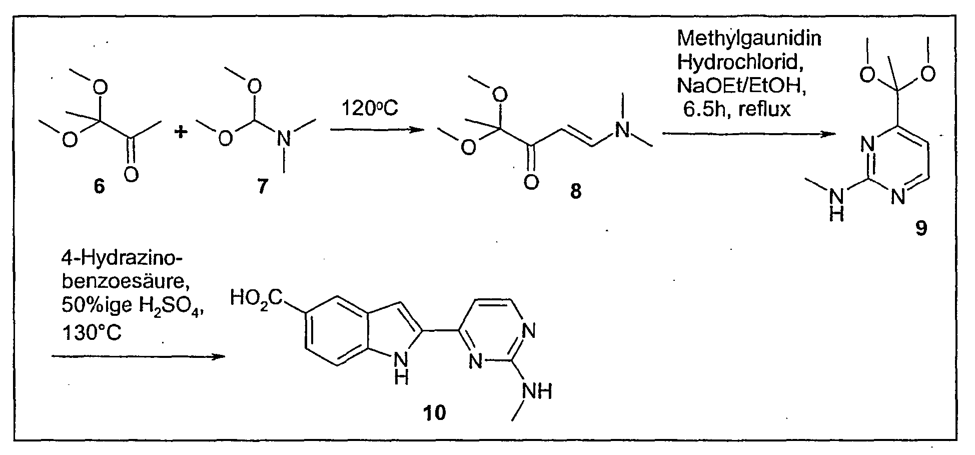

### 1-Dimethylamino-4,4-dimethoxy-pent-1-en-3-on (8)

100 g (0.76 mol) 3,3-Dimethoxy-2-butanon wurden mit 90,2 g N,N-Dimethylformamid-dimethylacetal (0.76 mol) bei 120°C 48 h gerührt. Das bei der Reaktion entstandene Methanol wurde kontinuierlich von der Reaktionslösung durch Destillation entfernt. Beim Erkalten der Lösung trat spontane Kristallisation ein, welche durch Zugabe von wenig Heptan zur Vollständigkeit gebracht wurde. Man erhielt so 128,24 g Rohprodukt **8** (Ausbeute 90%), welches ohne weitere Reinigung umgesetzt wurde.
Summenformel C₉H₁₇NO₃; M.W. = 187.24; MS (M+H) 188.2;
¹H NMR (DMSO-d₆) 1.22 (s, 3H), 2.80 (s, 3H), 3.10 (s, 9H), 5.39 (d, J = 15 Hz, 1H), 7.59 (d, J = 15 Hz, 1H).

### [4-(1,1-Dimethoxy-ethyl)-pyrimidin-2-yl]-methyl-amin (9)

1,22 g (53 mmol) Natrium wurden in 100 ml absoluten Ethanol gelöst. Dazu wurde unter Rühren 5,8 g (53 mmol) Methylguanidinhydrochlorid und 10 g (53 mmol) 1-Dimethylamino-4,4-dimethoxy-pent-1-en-3-on (**8**) gegeben und 4 h auf Siedehitze erwärmt. Zum Abbruch der Reaktion wurde das Ethanol evaporiert. Das so erhaltene Produkt **9** wurde ohne weitere Reinigung für die Folgereaktion eingesetzt. Ausbeute 11.5 g (58 mmol, quantitativ) Summenformel C₉H₁₅N₃O₂; M.W. = 197.24; MS (M+H) 198.2;
¹H NMR (DMSO-d₆) 1.45 (s, 3H), 2.78 (s, 3H), 3.10 (s, 6H), 6.75 (d, J = 3 Hz, 1H), 7.0 - 7.1(s(b), 1 H), 8.30 (d, J = 3 Hz, 1 H).

### 2-(2-Methylamino-pyrimidin-4-yl)-1 H-indol-5-carbonsäure (10)

In 150 ml 50%iger Schwefelsäure wurden bei Raumtemperatur 5 g (25 mmol) [4-(1,1-Dimethoxy-ethyl)-pyrimidin-2-yl]-methyl-amin (**9**) und 3.85 g 4-Hydrazinobenzoesäure unter Rühren gegeben und 4 h auf 130°C erhitzt. Das bei der Reaktion entstandene Methanol wurde kontinuierlich von der Reaktionslösung durch Destillation entfernt. Nach Abkühlen auf 10°C wurde die Reaktionsmischung auf 200 ml Eis gegossen und mit konzentrierter Natronlauge ein pH-Wert von etwa 5,5 eingestellt. Der dabei entstandene Niederschlag von Natriumsulfat und Produktgemisch wurde abfiltriert und der Filter-Rückstand wurde mehrmals mit Methanol extrahiert. Die vereinigten Methanol Extrakte wurde eingeengt und das Produkt durch Flash-Chromatographie (DCM/Methanol 9:1) gereinigt. Ausbeute: 0,76 g (11%) Summenformel C₁₄H₁₃N₄O₂; M.W. = 268.28; MS (M+H) 405.2; ¹H NMR (DMSO-d₆) 2.95 (s, 3H), 6.90 - 7.10 (s(b), 1H),7.18(d,J=3Hz, 1H), 7.4 (s, 1H), 7.58 (d, J = 4.5 Hz, 1H),7.80(d,J=4.5Hz, 1H), 8.30 (s, 1 H), 7.80 (d, J = 4.5 Hz, 1 H), 8.38 (d, J = 3 Hz, 1 H), 11.85 (s, 1H), 12.40 - 12.60 (s(b), 1 H).

### A.3.) Zusammenführung der Bausteine und Synthese von (2-(2-Methylamino-pyrimidin-4-yl)-1 H-indol-5-carbonsäure [(S)-2-diphenylamino-1-(5-oxo- 4,5-dihydro-[1,3,4]oxadiazol-2-yl)-ethyl]-amid (13))

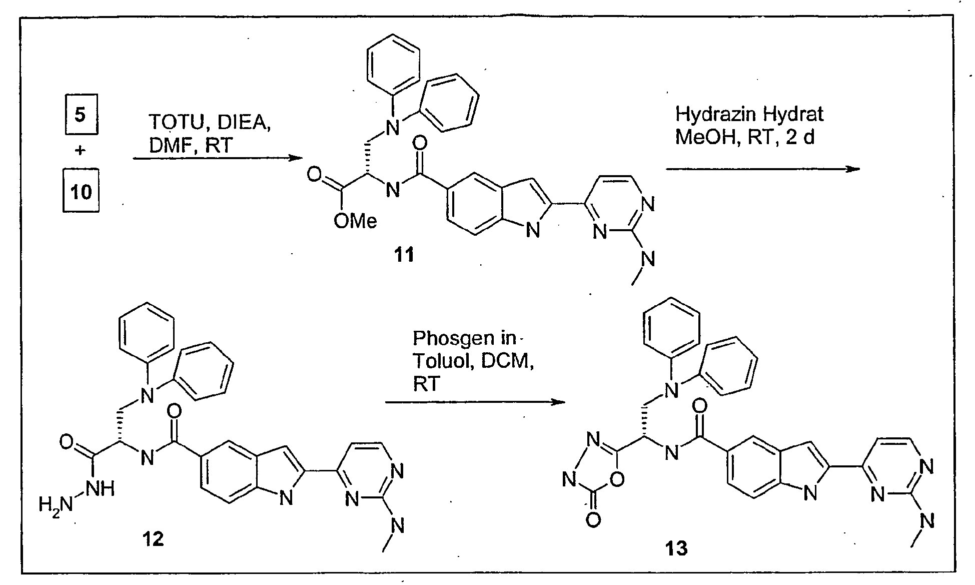

### 3-Diphenylamino-2-{[2-(2-methylamino-pyrimidin-4-yl)-1H-indol-5-carbonyl]- (S)-amino}-propionsäure (11)

5,0 g (18,64 mmol) 2-(2-Methylamino-pyrimidin-4-yl)-1H-indol-5-carbonsäure (**10**) wurden in 1,2 I DMF gelöst und nacheinander mit 7,9 g (24,08 mmol) TOTU und 7,9 ml (46,45 mmol) Ethyldiisopropylamin versetzt. Man rührte 20 min. bei 5°C und gab zu der Lösung 0,73 g (3,28 mmol) (S)-2-Benzyloxycarbonylamino-3-diphenylamino-propionsäure (**5**) hinzu. Nach 15 h Rühren engte man unter vermindertem Druck ein, nahm den Rückstand in n-Butanol auf und extrahierte die organische Phase zur Abtrennung von Nebenprodukten mit gesättigter Natriumhydrogencarbonatlösung. Nach dem Trocknen mit MgSO₄ und Einengen der organische Phase wurde der Methylester der Titelverbindung durch Flash-Chromatographie an Kieselgel (DCM:MeOH= 19:1) isoliert. Ausbeute: 4.3 g (98%)
Summenformel C₃₀H₂₈N₆O₃; M.W. = 520,22; MS (M+H) 521,3;
¹H NMR (DMSO-d₆) 2.95 (s(b), 3H), 3.60 (s, 3H), 4.19 - 4.58 (m, 2H), 4.85 (q,1 H), 6.90 - 7.10 (m, 7H), 7.18 (d, J = 3 Hz, 1 H), 7.25 - 7.40 (m, 5H), 7.50 (d, J = 4.5 Hz, 1 H), 7.65 (d, J = 4.5 Hz, 1 H), 8.05 (s, 1 H), 8.35 (d, J = 3 Hz, 1 H), 8.70 (d, J = 3.75 Hz, 1 H), 11.85 (s, 1 H).

### 2-(2-Methylamino-pyrimidin-4-yl)-1H-indol-5-carbonsäure ((S)-2-diphenylamin-1- hydrazin-carbonyl-ethyl)-amid (12)

1,0 g (1,92 mmol) 3-Diphenylamin-2-{[2-(2-methylamin-pyrimidin-4-yl)-1H-indol-5-carbonyl]-(S)-amino}- propionsäure (**11**) wurde in 10 ml Methanol gelöst, mit 0,48 g (9,95 mmol) Hydrazinhydrat versetzt und 15 h bei Raumtemperatur gerührt. Der Niederschlag des Produktes (0.3 g) wurde durch Filtration von der Mutterlauge getrennt. Aus der eingeengten Mutterlauge wurde durch Flash-Chromatographie an Kieselgel (DCM:MeOH= 19:1) weiteres Hydrazon **12** (0.1g) isoliert. Ausbeute: 0.4 g (40%)
Summenformel C₂₉H₂₈N₈O₂; M.W. = 520,6; MS (M+H) 521,4;
¹H NMR (DMSO-d₆) 2.95 (s(b), 3H), 4.02-4.58 (m, 2H), 4.4 (s, 2H), 4.85 (q,1H), 6.90-7.10 (m, 7H), 7.18 (d, J = 3 Hz, 1 H), 7.20 - 7.45 (m, 5H), 7.50 (d, J = 4.5 Hz, 1 H), 7.62 (d, J = 4.5 Hz, 1 H), 7.99 (s, 1 H), 8.25 (d, J = 3 Hz, 1 H), 8.35 (s(b), 1 H), 9.30 (s, 1 H), 11.70 (s, 1 H).

### 2-(2-Methylamin-pyrimidin-4-yl)-1 H-indol-5-carbonsäure [(S)-2-diphenylamin-1-(5-oxo- 4,5-dihydro-[1,3,4]oxadiazol-2-yl)-ethyl]-amid (13)

200 mg (0.384 mmol) 2-(2-Methylamin-pyrimidin-4-yl)-1H-indol-5-carbonsäure ((S)-2-diphenylamin-1-hydrazincarbonyl-ethyl)-amid (**12**) wurden in 20 ml Methylenchlorid suspendiert und bei 0°C wurde eine 20%ige Phosgen Lösung in Toluol (0.398 mmol) unter Rühren zugetropft. Es wurde weitere 15 h bei Raumtemperatur gerührt und das Lösungsmittel eingeengt. Das Oxadiazolon **13** wurde anschließend durch Flash-Chromatographie an Kieselgel (DCM:MeOH= 9 :1) isoliert. Ausbeute: 160 mg (76%)
Summenformel C₃₀H₂₆N₈O₃; M.W. = 546,6; MS (M+H) 547.3;
¹H NMR (DMSO-d₆) 2.95 (s(b), 3H), 4.02 - 4.58 (m, 2H), 4.85 (q,1H), 6.90 - 7.10 (m, 7H), 7.15 (d, J = 3 Hz, 1 H), 7.20 - 7.40 (m, 6H), 7.52 (d, J = 4.5 Hz, 1 H), 7.68 (d, J = 4.5 Hz, 1 H), 8.10 (s, 1 H), 8.92 (d, J = 3 Hz, 1 H), 11.78 (s, 1 H), 12.15 - 12.40 (s(b), 1 H).

### B.) Beispiel Benzimidazol IkB Kinase Inhibitor

### B.1.) Synthese der Aminosäure ((S)-2-Amino-3-diphenylamino-propionic acid methyl ester (5)) wie unter A.1. beschrieben

### B.2.) Synthese des heterozyklischen Grundkörpers (2-(2-Methylamino-pyrimidin-4-yl)-1 H-benzimidazol-5-carbonsäure (19))

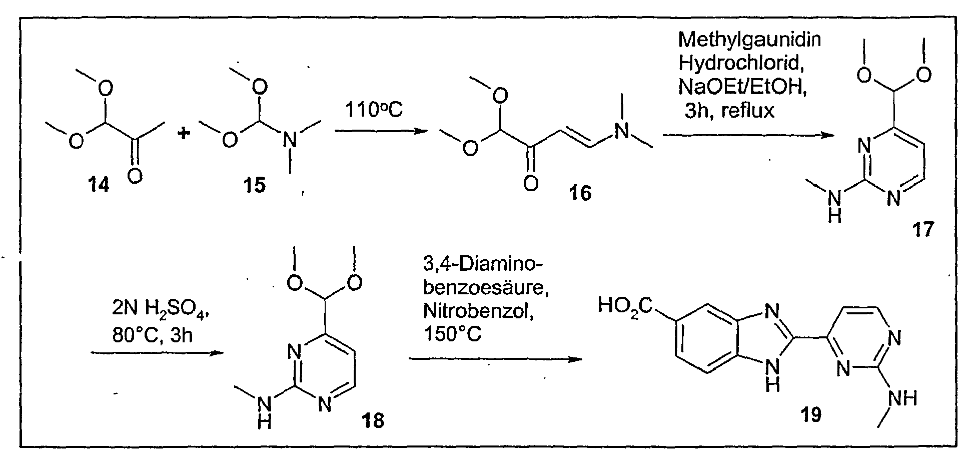

### 4-Dimethylamin-1,1-dimethoxy-but-3-en-2-on (16)

300 g (307 ml, 2.54 mol) Methylglyoxaldimethylacetal wurden mit 303 g (337 ml, 2.54 mol) N,N-Dimethylformamid-dimethylacetal bei 110°C 4 Stunden (h) gerührt. Das bei der Reaktion entstandene Methanol wurde kontinuierlich von der Reaktionslösung durch Destillation entfernt. Nach dem Erkalten wurde die Lösung mit Heptan extrahiert und die Lösungsmittel evaporiert. Man erhielt so 303 g Rohprodukt **16** (Ausbeute 70%) welches ohne weitere Reinigung umgesetzt wurde.
Summenformel C₈H₁₅NO₃; M.W. = 173.21; MS (M+H) 174.1;
¹H NMR (DMSO-d₆) 2.10 (s, 1H), 2.80 (s, 3H), 3.10 (s, 3H), 3.25 (s, 3H), 3.3 (s, 3H), 4.42 (s, 1H), 5.19 (d (b), J = 12.8 Hz, 1H), 7.60 (d, J = 15 Hz, 1H).

### (4-Dimethoxymethyl-pyrimidin-2-yl)-methyl-amin (17)

0,33 g (14,4 mmol) Natrium wurden in 50 ml absoluten Ethanol aufgelöst. Dazu wurde unter Rühren 1,57 g (14,4 mmol) Methylguanidinhydrochlorid und 2.48 g (14.4 mmol 4-Dimethylamin-1,1-dimethoxy-but-3-en-2-on (**16**) gegeben und 3 h auf Siedehitze erwärmt. Zum Abbruch der Reaktion wurde das Ethanol evaporiert. Das so erhaltene Produkt **17** wurde ohne weitere Reinigung eingesetzt. Ausbeute 2.6 g (quantitativ).
Summenformel C₈H₁₃N₃O₂; M.W. = 183.21 ; MS (M+H) 184.1;
¹H NMR (DMSO-d₆) 2.78 (s, 6H), 3.10 (s, 3H), 5.02 (s,1H), 6.62 (d, J = 3 Hz, 1H), 8.30 (d, J = 3 Hz, 1H).

### 2-Methylamin-pyrimidin-4-carbaldehyd (18)

10 g (54mmol)(4-Dimethoxymethyl-pyrimidin-2-yl)-methyl-amin (**17**) wurden in 54 ml 2N Schwefelsäure aufgelöst und unter Rühren 3 h auf 80°C erwärmt. Nach dem Erkalten der Reaktion wurde die Reaktionslösung vorsichtig mit festen Na₂CO₃ auf pH von etwa 9 gebracht und mit Ethanol 3 mal extrahiert. Die vereinigten getrockneten Extrakte ergaben nach Evaporierung des Lösungsmittels den Titelaldehyd 18 in 60%iger Ausbeute (4.47g) Summenformel C₆H₇N₃O; M.W. = 137.12 ; MS (M+H) 138.2;
¹H NMR (DMSO-d₆) 2.60-2.80 (s(b), 3H), 6.95 (d, J = 3 Hz, 1 H), 7.40-7.60 (s(b), 1 H), 8.55 (d, J = 3 Hz, 1 H).

### 2-(2-Methylamin-pyrimidin-4-yl)-1H-benzoimidazol-5-carbonsäure (19)

4.3 g (31,3 mmol) Methylamin-pyrimidin-4-carbaldehyd (**18**) und 4.8 g (31,1 mmol) 3,4-Diamino-benzoesäure wurden in 300 ml Nitrobenzol 2 h auf 150°C erhitzt. Nach Abkühlen auf 0°C wurde der Niederschlag des Benzimidazols durch Filtration von dem Nitrobenzol getrennt und das Produkt durch Flash-Chromatographie (DCM/Methanol 4 :1) gereinigt. Ausbeute: 2.66 g (32%)
Summenformel C₁₃H₁₁N₅O₂; M.W. = 269.28; MS(M+H) 270.2;
¹H NMR (DMSO-d₆) 2.95 (s, 3H), 7.50 (d, J = 3 Hz, 1H), 7.75 (d, J = 4.5 Hz, 1 H), 7.90 (d, J = 4.5 Hz, 1 H), 8.35 (s, 1 H), 8.55 (d, J = 3 Hz, 1 H), 8.70 - 9.05 (s(b), 1 H).

### .3.) Zusammenführung der Bausteine und Synthese von 2-(2-Methylamino-pyrimidin-4-yl)-1H-benzoimidazol-5-carbonsäure ((S)1-carbamoyl-2- diphenylamin-ethyl)-amid (22)

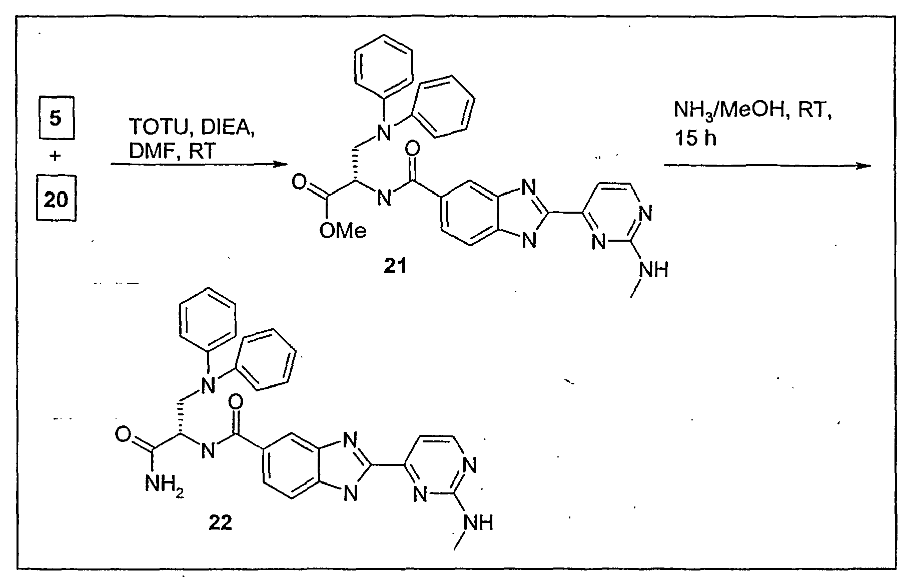

### 3-Diphenylamino-2-{[2-(2-methylamino-pyrimidin-4-yl)-1H-benzimidazol-5-carbonyl]- (S)-amino}- propionsäure (21)

2.6 g (9.6 mmol) 2-(2-Methylamin-pyrimidin-4-yl)-1H-benzimidazol-5-carbonsäure (**20**) wurde in 300 ml DMF gelöst und nacheinander mit 3.17 g (9.6 mmol) TOTU und 1.6 ml (11.6 mmol) Ethyldiisopropylamin versetzt. Man rührte 20 min bei 5°C und gab zu der Lösung 2.6 g (9.6 mmol) (S)-2-Benzyloxycarbonylamin-3-diphenylamin-propionsäure (**5**) hinzu. Nach 16 h Rühren engte man unter vermindertem Druck ein, anschließend wurde der Methylester **21** durch Flash-Chromatographie an Kieselgel (DCM:MeOH= 9:1) isoliert. Ausbeute: 1.61 g (32%) Summenformel C₂₉H₂₇N₇O₃; M.W. = 521,58; MS (M+H) 522.3;
¹H NMR (DMSO-d₆) 2.95 (s(b), 3H), 3.60 (s, 3H), 4.19-4.40 (m, 2H), 4.90 (q,1H), 6.90-7.10 (m, 6H), 7.25 - 7.35 (m, 6H), 7.40 (d, J = 4.5 Hz, 1 H), 7.60 - 7.80 (d(b) 1 H), 8.05 - 8.25 (d(b), 1 H), 8.45 (d, J = 3 Hz, 1 H), 8.90 (s(b), 1 H), 11.85 (s(b), 1H).

### 2-(2-Methylamino-pyrimidin-4-yl)-1 H-benzoimidazol-5-carbonsäure ((S)1-carbamoyl-2-diphenylamin-ethyl)-amid (22)

50 ml Methanol (absolut) wurden bei 0°C mit Ammoniak gesättigt. Dazu wurde 0.5 g (0.959 mmol) 3-Diphenylamin-2-{[2-(2-methylamin-pyrimidin-4-yl)-1H-benzimidazol-5-carbonyl]- (S)-amin}- propionsäure (**21**) gegeben und 24 h bei Raumtemperatur gerührt. Nach Evaporation des Lösungsmittels und überschüssigen Ammoniaks wurde durch Flash-Chromatographie an Kieselgel (DCM:MeOH= 19:1) das Amid **22** isoliert. Ausbeute: 0.43 g (89%)
Summenformel C₂₉H₂₈N₈O₂; M.W. = 506,57; MS (M+H) 507.2;
¹H NMR(DMSO-d₆)2.95 (s(b), 3H), 4.02 - 4.35 (m, 2H), 4.85 (q,1 H), 6.80 - 7.10 (m, 6H), 7.15 - 7.25 (m, 5H), 7.40 (d, J = 4.5 Hz, 1 H), 7.58 (s(b), 1 H), 7.68 (s(b), 1 H), 8.06 - 8.19 (d(b), 1 H), 8.40 - 8.58 (m, 2H), 13.10 (s, 1 H).

### Pharmakologische Beispiele

### IκB-Kinase ELISA:

Die Aktivität der IκB-Kinase wurde mit einem ELISA, bestehend aus einem biotiniliertem Substratpeptid, welches die Aminosäuresequenz im Protein IκB von Serin 32 bis 36 enthält, und einem spezifischen poly- oder monoklonalen Antikörper (z.B. von New England Biolabs, Beverly, MA, USA, Kat.: 9240), der nur an die phosphorylierte Form des Peptids IκB bindet, bestimmt. Dieser Komplex wurde an einer antikörperbindenden Platte (Protein A beschichtet) immobilisiert und mit einem Konjugat aus einem biotinbindendem Protein und HRP (z.B. Streptavidin-HRP) detektiert. Die Aktivität konnte an Hand einer Standardkurve mit Substratphosphopeptid quantifiziert werden.

### Durchführung:

Zur Gewinnung des Kinasekomplexes wurden 10 ml HeLa S3-Zellextrakt S100 mit 40 ml 50mM HEPES, pH 7,5, verdünnt, auf 40% Ammoniumsulfat gebracht und auf Eis 30 Minuten inkubiert. Das präzipitierte Pellet wurde in 5 ml SEC Puffer (50 mM HEPES, pH 7,5, 1 mM DTT, 0,5 mM EDTA, 10 mM 2-Glyzerophosphat) gelöst, bei 20,000 x g für 15 Minuten zentrifugiert und durch einen 0,22 µm Filter filtriert. Die Probe wurde auf eine 320 ml Superose-6 FPLC Säule (Amersham Pharmacia Biotech AB, Uppsala, Schweden) aufgetragen, die mit SEC Puffer äquilibriert war und mit einer Flußrate von 2 ml/min bei 4 °C betrieben wurde. Die Fraktionen, die bei der Laufzeit des 670 kDa Molekulargewichtstandards lagen, wurden für die Aktivierung vereinigt. Die Aktivierung wurde durch eine 45-minütige Inkubation mit 100 nM MEKK1Δ, 250 µM MgATP, 10 mM MaCl₂, 5 mM Dithiothreitol (DTT), 10 mM 2-Glyzerophosphat, 2,5 µM Microcystin-LR bei 37°C erreicht. Das aktivierte Enzym wurde bei -80 °C gelagert. Die in DMSO gelösten Testsubstanzen (2 µl) wurden 30 Minuten bei 25°C mit 43 µl aktiviertem Enzym (1:25 verdünnt in Reaktionspuffer 50 mM HEPES, pH 7,5, 10 mM MgCl₂, 5 mM DTT, 10 mM β-Glycerophosphat, 2,5 µM Microcystin-LR) vorinkubiert. Dann wurden 5 µl Substratpeptid (Biotin-(CH₂)₆-DRHDSGLDSMKD-CONH₂) (200 µM) dazugegeben, eine Stunde inkubiert und mit 150 µl 50 mM HEPES pH 7,5, 0.1% BSA, 50 mM EDTA, Antikörper [1:200] abgestoppt. 100 µl des abgestoppten Reaktionsgemisches bzw. einer Standardphosphopeptidverdünnungsreihe (Biotin-(CH₂)₆-DRHDS[PO₃]GLDSMKD-CONH₂) wurden dann in eine Protein-A Platte (Pierce Chemical Co., Rockford, IL, USA) überführt und 2 Stunden unter Schütteln inkubiert. Nach 3 Waschschritten mit PBS, wurden 100 µl 0,5 µg/ml Streptavidin-HRP (horseradish peroxidase) (verdünnt in 50 mM HEPES/ 0,1% BSA) für 30 Minuten hinzugegeben. Nach 5 Waschschritten mit PBS, wurden 100 µL TMB-Substrat (Kirkegaard & Perry Laboratories, Gaithersburg, MD, USA) hinzugegeben und die Farbentwicktung durch Zugabe von 100 µL 0,18 M Schwefelsäure abgestoppt. Die Absorption wurde bei 450 nm gemessen. Die Standardkurve wurde durch lineare Regression entsprechend einer 4-Parameter Dosis-Wirkungsbeziehung erzeugt. An Hand dieser Standardkurve wurde die Enzymaktivität bzw. deren Inhibition durch die Testsubstanzen quantifiziert.

Die IC₅₀ für 2-(2-Methylamin-pyrimidin-4-yl)-1 H-indol-5-carbonsäure [(S)-2-diphenylamin-1-(5-oxo- 4,5-dihydro-[1,3,4]oxadiazol-2-yl)-ethyl]-amid betrug 0,050 µM. Die 1C₅₀ für 2-(2-Methylamino-pyrimidin-4-yl)-1 H-benzoimidazol-5-carbonsäure ((S)1-carbamoyl-2- diphenylamin-ethyl)-amid betrug 0,045 µM.

### Schmerzassay

Die analgetische und anti-nozizeptive Wirksamkeit der Verbindung 2-(2-Methylamin-pyrimidin-4-yl)-1H-indol-5-carbonsäure [(S)-2-diphenylamin-1-(5-oxo-4,5-dihydro-[1,3,4]oxadiazol-2-yl)-ethyl]-amid - im folgenden Verbindung 13 genannt - wurde in den zwei folgenden Modellen nachgewiesen:
1. Modell: Zymosan-induzierte Pfotenentzündung bei der Ratte;
   Parameter: Pfotenwegziehzeit oder Pfotenwegziehschwelle bei thermischer oder mechanischer Stimulation der Hinterpfote.
2. Modell: Kaolin/Carrageenan induzierte Kniegelenksentzündung bei der Ratte;
   Parameter: Reaktion spinaler Neurone bei Druckreizung des Knies.

### Modell 1

Versuchsdurchführung: In Kurzzeitnarkose mit Isofluran wurde dem Versuchstier subkutan 1 mg Zymosan (als Suspension in 100 µl PBS (mit Phosphat gepufferte Salzlösung) in die Mitte der Plantarseite einer Hinterpfote injiziert. Im Anschluss daran wurde mit zwei verschiedenen Verhaltenstests die Entwicklung einer Hyperalgesie quantitativ bestimmt.
a) Bestimmung der Pfotenwegziehzeit bei thermischer Stimulation (Hargreaves Test). Das Versuchstier wurde in eine durchsichtige Plastikkammer mit einem Glasboden gesetzt. Sobald sich das Versuchstier nach der Erkundungsphase (etwa 5 min) nicht mehr bewegte, wurde eine Infrarotlichtquelle direkt unterhalb der zu stimulierenden Hinterpfote positioniert und angeschaltet. Die Lampe strahlte fokussiertes Infrarotlicht steigender Intensität ab, so dass die Hauttemperatur der Hinterpfote annähernd linear anstieg. Sobald das Tier die Pfote wegzog, schaltete sich die Lampe ab. Die Pfotentemperatur zum Zeitpunkt des Wegziehens ist für das Tier gerade eben unangenehm, man spricht von thermischer Schmerzschwelle.
b) Bestimmung der Pfotenwegziehschwelle bei mechanischer Stimulation (von Frey Test) Das Versuchstier wurde in eine durchsichtige Plastikkammer gesetzt, dessen Boden aus Maschendraht bestand. Mit Hilfe kalibrierter Nylonfäden, sogenannter von Frey Haare, wurde punktförmiger Druck definierter Stärke erzeugt. Der schwächste Druckreiz, während dessen das Tier die Pfote wegzog, bestimmt die mechanische Schmerzschwelle.

Etwa eine halbe Stunde vor und zu verschiedenen Zeitpunkten nach der Zymosan Injektion wurden die thermische und mechanische Schmerzschwellen an der rechten und linken Hinterpfote bestimmt (siehe Tabellen 1,2). Berechnet wurde dann die Abnahme der ipsilateralen Schmerzschwelle, ausgedrückt in % der kontralateralen Schmerzschwelle (siehe Tabellen 1,2). Je stärker diese Abnahme ist, umso ausgeprägter ist die Hyperalgesie.

Die Zymosan Injektion induzierte in einer Kontrollgruppe eine ausgeprägte mechanische und thermische Hyperalgesie (s. Kontrolldaten in Tab. 1 und 2). Etwa 15 Minuten vor und 2,5 und 5,5 Stunden nach Zymosan Injektion wurde in einer weiteren Gruppe von Tieren unter Kurzzeitnarkose mit Isofluran die obengenannte Verbindung 13 intraperitoneal (i.p.) injiziert (je 30 mg/kg in Polyethylenglycol-Wasser Gemisch (PEG-Wasser 1:1). Die thermische Hyperalgesie war in diesen Tieren ab zwei Stunden nach Zymosan Injektion geringer ausgeprägt als in der Kontrollgruppe, nach der dritten Substanzgabe war überhaupt kein Seitenunterschied in der Pfotenwegziehzeit mehr zu finden (Tab. 1). Dieser Effekt hielt auch über 18 Std. nach der letzten Substanzgabe noch an.

Die mechanische Hyperalgesie wurde durch die Verbindung 13 ebenfalls signifikant reduziert. Der Effekt setzte 1 Stunde nach Zymosan Injektion ein und hielt auch 18 Std. nach der letzten Substanzgabe noch an (siehe Tab. 2).

Die Wirksamkeit der Verbindung 13 ist in beiden Testmodellen sehr stark. Vergleichsdaten aus einer früher durchgeführten Studie zeigen, dass die Verbindung 13 die thermische Hyperalgesie erheblich stärker reduziert als das NSAID Diclofenac.

**Tabelle 1: Veränderung der Pfotenwegziehzeit (%)**

| Zeit (h) nach Zymosan-Injektion (0) | Mittelwert Verbindung 13 | SD Verbindung 13 | Mittelwert Kontrolle | SD Kontrolle |
|---|---|---|---|---|
| Baseline -0,5 | 0,0 | 0,0 | 0,0 | 0,0 |
| 0,5 | -16,6 | 6,6 | -21,4 | 6,3 |
| 1 | -31,3 | 14,1 | -28,8 | 11,6 |
| 2 | -30,2 | 15,4 | -44,8 | 19,1 |
| 3 | -15,3 | 5,3 | -49,2 | 17,9 |
| 4 | -16,0 | 11,5 | -50,6 | 23,0 |
| 5 | -9,7 | 18,6 | -46,6 | 24,8 |
| 6 | 5,0 | 2,6 | -38,4 | 17,6 |
| 7 | 3,4 | 5,8 | -29,9 | 22,1 |
| 24 | -3,8 | 7,0 | -46,1 | 18,4 |

**Tabelle 2:**

| Veränderung der Pfotenwegziehschwelle (%) | | | | |
|---|---|---|---|---|
| Zeit (h) nach Zymosan-Injektion (0) | Mittelwert Verbindung 13 | SD Verbindung 13 | Mittelwert Kontrolle | SD Kontrolle |
| Baseline -0,5 | 0,0 | 0,0 | 0,0 | 0,0 |
| 0,5 | -37,4 | 6,6 | -48,9 | 31,3 |
| 1 | -43,1 | 20,5 | -66,0 | 23,2 |
| 2 | -36,0 | 17,8 | -71,8 | 26,0 |
| 3 | -35,1 | 13,1 | -60,5 | 20,2 |
| 4 | -46,7 | 11,9 | -64,3 | 18,2 |
| 5 | -40,6 | 14,0 | -55,5 | 25,8 |
| 6 | -33,1 | 23,3 | -57,3 | 18,0 |
| 7 | -44,7 | 21,5 | -47,1 | 23,9 |
| 24 | -9,7 | 26,6 | -41,5 | 17,3 |

### Modell 2,

Versuchsdurchführung: Bei Ratten wurde in Trapanal-Narkose der Wirbelkanal eröffnet und Rückenmarksneurone, die "Schmerzimpulse" aus dem Kniegelenk verarbeiteten, identifiziert. Nach Identifizierung wurde eine Langzeitableitung durchgeführt, in der die Aktivität der Nervenzelle vor und während der Entwicklung einer akuten Entzündung im Kniegelenk registriert wurde. Dazu wurden in einer Kontrollperiode vor Induktion der Entzündung und nach Induktion der Entzündung für mehrere Stunden die Antworten auf nicht noxische und noxische Reizung am Kniegelenk gemessen.

Die akute Entzündung wurde durch die intraartikuläre Injektion einer Kaolin und Carrageenan Suspension (etwa 150 µl) induziert. In Kontrollexperimenten wurde nur das Vehikel auf die Rückenmarksoberfläche aufgebracht, um die Entwicklung der Übererregbarkeit unter Kontrollbedingungen darzustellen. In der Regel fand diese Entwicklung der Übererregbarkeit innerhalb von 2 bis 4 Stunden statt, und sie äußerte sich in einer starken Zunahme der Antworten auf nicht noxische und noxische Reizung des Kniegelenks (Tab. 3). In den Experimenten, in denen die obengenannte Verbindung 13 appliziert wurde, wurde die Substanz etwa 30 Minuten vor Induktion der Entzündung auf das Rückenmark gegeben (etwa 30 µl einer 10 µM Lösung). Dann wurden die Antworten der Zelle auf nichtnoxische und noxische Reizung wie in den Kontrollexperimenten weiter verfolgt.

Der Vergleich der Veränderungen der Antworten in den beiden Gruppen zeigt, dass die Verbindung 13 die Entwicklung der spinalen Übererregbarkeit gegenüber den Kontrollen beinahe vollständig unterdrückt hat (Tab. 3). Die Wirkung der Verbindung 13 auf die Antworten auf noxische Kniegelenksstimulation war insgesamt stärker ausgeprägt als die Wirkung von Indomethacin, wie ein Vergleich mit publizierten Daten aus einer früheren Studie ergab.

**Tabelle 3: Neuronale Antworten vor und während Kniegelenksentzündung (imp/15s Noxische Reizung am Kniegelenk**

| Zeit (min) nach K/C-Injektion | Mittelwert Verbindung 13 | SEM Verbindung 13 | Mittelwert Kontrolle | SEM Kontrolle |
|---|---|---|---|---|
| Baseline | 0,8 | 29,9 | 0 | 0 |
| 30-60 | 62,3 | 49,3 | 161,6 | 43,7 |
| 60-120 | 26,9 | 35 | 458,1 | 125,4 |
| 120-180 | 8,5 | 58,9 | 544,2 | 140,0 |
| 180-240 | 19,5 | 59,9 | 616,3 | 174,7 |
| | | Nicht-noxische Reizung am Kniegelenk | | |

| Zeit (min) nach K/C-Injektion (0) | Mittelwert Verbindung 13 | SEM Verbindung 13 | Mittelwert Kontrolle | SEM Kontrolle |
|---|---|---|---|---|
| Baseline | 0,92 | 16,90 | 0 | 0 |
| 30-60 | 8,66 | 23,76 | 21,4 | 11,9 |
| 60-120 | 2,71 | 25,94 | 74,6 | 38,3 |
| 120-180 | 11,16 | 24,22 | 105,7 | 39,0 |
| 180-240 | 39,78 | 25,09 | 149,7 | 44,3 |

Ferner wurde die Wirkung von 2-(2-Methylamino-pyrimidin-4-yl)-1 H-benzoimidazol-5-carbonsäure ((S)1-carbamoyl-2- diphenylamin-ethyl)-amid - im folgenden Verbindung 22 genannt- im Modell 2 getestet

Kontrolldaten: s. Tabelle 3

**Tabelle 4: Neuronale Antworten vor und während Kniegelenksentzündung (imp/15s)**

| | | |
|---|---|---|
| | Noxische Reizung am Kniegelenk | |

| Zeit (min) nach K/C-Injektion | Verbindung 22 Versuch 1 | Verbindung 22 Versuch 2 |
|---|---|---|
| Baseline | 0 | 0 |
| 30-60 | -109,1 | -9,2 |
| 60-120 | -101,1 | |
| 120-180 | -37,8 | 60 |
| 180-240 | | 96,7 |
| | Nicht-noxische Reizung am Kniegelenk | |

| Zeit (min) nach K/C-Injektion (0) | Verbindung 22 Versuch 1 | Verbindung 22 Versuch 2 |
|---|---|---|
| Baseline | 0 | 0 |
| 30-60 | -34,1 | -30,6 |
| 60-120 | -37,2 | |
| 120-180 | -32,1 | 50,3 |
| 180-240 | | 68,7 |

Die Daten belegen die gute Wirkung der Verbindung 22 im Modell 2.

### 3. Modell: Zymosan-induzierte Pfotenentzündung bei der Maus;

Parameter: Pfotenwegziehzeit bei thermischer Stimulation der Hinterpfote. Versuchsdurchführung: In Kurzzeitnarkose mit Isofluran wurde dem Versuchstier 25 µl einer Suspension, die 50 mg/ml Zymosan enthielt, in die rechte Hinterpfote injiziert. Dann wurde die Entwicklung einer Hyperalgesie quantitativ wie folgt bestimmt:

Bestimmung der Pfotenwegziehzeit bei thermischer Stimulation (Hargreaves Test; s.o.). Das Versuchstier wurde in eine durchsichtige Plastikkammer mit einem Glasboden gesetzt. Sobald sich das Versuchstier nach der Erkundungsphase (etwa 5 min) nicht mehr bewegte, wurde eine Infrarotlichtquelle direkt unterhalb der zu stimulierenden Hinterpfote positioniert und angeschaltet. Die Lampe strahlte fokussiertes Infrarotlicht steigender Intensität ab, so dass die Hauttemperatur der Hinterpfote annähernd linear anstieg. Sobald das Tier die Pfote wegzog, schaltete sich die Lampe ab. Die Pfotentemperatur zum Zeitpunkt des Wegziehens ist für das Tier gerade eben unangenehm, man spricht von thermischer Schmerzschwelle.

Kurz vor Zymosan-Injektion und für 7 bis 14 Tage nach Injektion wurde einmal täglich die thermische Schmerzschwelle an der rechten und linken Hinterpfote bestimmt. Anschließend wurde als Maß für die Hyperalgesie das Integral der Fläche, die aus den Kurven der Pfotenwegziehzeiten der entzündeten und der nicht-entzündeten Pfote gebildet wurde, bestimmt (AUC, area between the curves, s. Tabellen 5 und 6). Je größer dieser Wert ist, umso ausgeprägter ist die Hyperalgesie, und je kleiner der Wert bei Tieren, die die Substanz erhalten, desto stärker ist der Therapieerfolg.

In einer 7-Tage Studie induzierte die Zymosan Injektion in einer Kontrollgruppe eine ausgeprägte thermische Hyperalgesie (s. Vehikel, Tab. 5). In den anderen Gruppen wurde die Substanzgabe einen Tag nach Zymosan Injektion, nachdem schon eine deutliche thermische Hyperalgesie entstanden war, begonnen. Die Verbindung 13 wurde dann zweimal täglich für 7 Tage oral verabreicht, und zwar je 25 oder 75 mg/kg in HEC/Lipofundin (1 % HEC in Lipofundin). Die Auswertung der Pfotenwegziehzeiten während des Gesamtzeitraums der Studie (7 Tage) ergab, dass die AUC unter Substanzgabe dosisabhängig abnahm. Bei Dosen ab 8.3 mg/kg Einzeldosis war der Therapieerfolg signifikant gegenüber der Vehikelgruppe (Tab. 5). Die Wirksamkeit der Verbindung 13 in dem Testmodell ist sehr stark. In einer parallel mitgeführten Gruppe von Tieren wurde Paracetamol in einer sehr hohen Dosis ebenfalls zweimal am Tag verabreicht. Die Verbindung 13 reduzierte die thermische Hyperalgesie stärker als Paracetamol (Tab. 5).

**Tabelle 5 Thermische Hyperalgesie während sieben Tagen nach Zymosan Injektion**

| | AUC Mittelwert [Maß für Hyperalgesie] | Standardfehler des arithmetischen Mittels (SEM) | Anzahl der Tiere pro Gruppe | Statistischer Unterschied gegenüber Vehikel |
|---|---|---|---|---|
| Vehikel | 45.1 | 1.5 | 8 | |
| Paracetamol 200 mg/kg | 24.6 | 4.1 | 8 | ja |
| Verbindung 13 2.8 mg/kg | 40.4 | 2.4 | 8 | nein |
| Verbindung 13 8.3 mg/kg | 32.3 | 2.2 | 8 | ja |
| Verbindung 13 25 mg/kg | 19.4 | 2.9 | 8 | ja |
| Verbindung 13 75 mg/kg | 17.4 | 2.6 | 8 | ja |

In einer weiteren Studie an Mäusen wurde die Wirksamkeit der Verbindung 13 mit dem spezifischen COX-2 Inhibitor Celecoxib verglichen. Zymosan Injektion und Dosierschema waren identisch mit dem in der zuvor beschriebenen Studie. Einziger Unterschied war, dass die weitere Studie über 14 Tage lief.

Durch die Verbindung 13 konnte wiederum dosisabhängig die thermische Hyperalgesie reduziert werden (Tab. 6). Dabei waren die Effekte der Verbindung 13 und Celetoxib in der hohen Dosierung gleich stark (Tab. 6)

**Tabelle 6 Thermische Hyperalgsie während 14 Tagen nach Zymosan Injektion**

| | AUC Mittelwert [Maß für Hyperalgesie] | Standardfehler des arithmetischen Mittels (SEM) | Anzahl der Tiere pro Gruppe | Statistischer Unterschied gegenüber Vehikel |
|---|---|---|---|---|
| Vehikel | 90.0 | 5.1 | 8 | |
| Celecoxib 8.3 mg/kg | 79.9 | 5.9 | 5 | nein |
| Celecoxib 25 mg/kg | 51.5 | 3.7 | 9 | ja |
| Verbindung 13 8.3 mg/kg | 64.5 | 5.0 | 5 | ja |
| Verbindung 13 25 mg/kg | 47.6 | 4.4 | 9 | ja |

### 4. Modell: Zymosan-induzierte Pfotenentzündung bei der Maus; Parameter: Spontane Laufleistung im Laufrad.

Das Versuchstier hat in seinem Haltungskäfig Zugang zu einem Laufrad, dessen Umdrehungen elektronisch registriert werden. Die C57/B6 Mäuse benutzen in den Nachtstunden freiwillig das Laufrad und legen im Mittel nach einer einwöchigen Gewöhnungsphase 4100 Meter/Nacht zurück. Nach Zymosan Injektion ist die nächtliche Laufstrecke verkürzt. Diese Abnahme der Laufleistung gilt als Parameter für entzündungsschmerzbedingte Funktionseinschränkung. Versuchsdurchführung: Die Laufstrecke/24 Std. wurde nach einer Eingewöhnungsphase eine Woche lang zur Bestimmung der Baseline gemessen. Dann wurde in Kurzzeitnarkose mit Isofluran dem Versuchstier 25 µl einer Suspension, die 50 mg/m Zymosan enthielt, in die rechte Hinterpfote injiziert. Anschließend wurde Laufstrecke/24 Std. während der nächsten sieben Tage ermittelt. In der Auswertung wurde die Fläche unter der Kurve der Laufstreckenwerte bestimmt (AUC, Tab. 7): je kleiner die AUC, desto geringer die Laufleistung in der Woche nach Zymosan Injektion. Die Verbindung 13 wurde zweimal täglich für 7 Tage oral verabreicht, und zwar je 25 oder 75 mg/kg in HEC/Lipofundin (1% HEC in Lipofundin). Die Substanzgabe wurde am Tag 1 nach Zymosan Injektion begonnen.

In einer Studie wurde die Wirkung der Verbindung 13 im Vergleich zu Paracetamol auf die Laufleistung nach Zymosan Injektion bestimmt. Es zeigte sich eine dosisabhängige Steigerung der Laufstrecke, die für die beiden höheren Dosierungen signifikant gegenüber der Vehikelgruppe war (Tab. 7). Mit Paracetamol in einer extrem hohen Dosis (ebenfalls 2x täglich) wurde im Gegensatz hierzu keine Verbesserung gegenüber der Vehikelgruppe erzielt (Tab. 7).

**Tabelle 7 Laufradaktivität während sieben Tage nach Zymosan Injektion**

| | AUC Mittelwert | Standardfehler des arithmetischen Mittels (SEM) | Anzahl der Tiere pro Gruppe | Statistischer Unterschied gegenüber Vehikel |
|---|---|---|---|---|
| Vehikel | 108.8 | 12.5 | 8 | |
| Paracetamol 200 mg/kg | 187.2 | 42.7 | 8 | nein |
| Verbindung 13 2.8 mg/kg | 131.1 | 23.3 | 8 | nein |
| Verbindung 13 8.3 mg/kg | 142.1 | 29.1 | 8 | nein |
| Verbindung 13 25 mg/kg | 216.7 | 58.5 | 8 | ja |
| Verbindung 13 75 mg/kg | 251.7 | 41.9 | 8 | ja |

## Patentansprüche

1. Verwendung der Verbindung der Formel la, 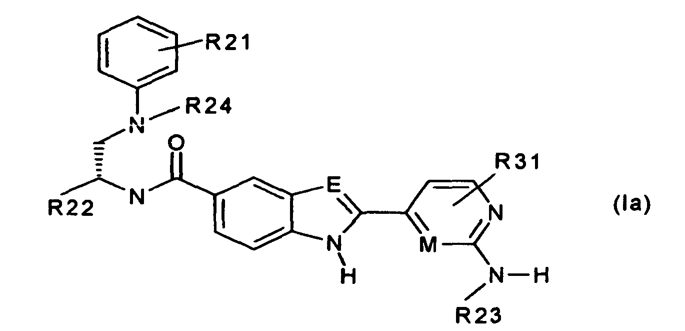 und/oder eine stereoisomere Form der Verbindung der Formel la und/oder ein physiologisch verträgliches Salz der Verbindung der Formel la,
zur Herstellung von Arzneimitteln zur Behandlung von Schmerzen, wobei E und M gleich oder verschieden sind und unabhängig voneinander für N-Atom oder CH stehen,
R21 und R31 gleich oder verschieden sind und unabhängig voneinander für
1. Wasserstoffatom,
2. Halogen,
3. -(C₁-C₄)-Alkyl,
4. -CN,
5. -CF₃,
6. -OR¹⁵, worin R¹⁵ für Wasserstoffatom oder -(C₁-C₄)-Alkyl steht,
7. -N(R¹⁵)-R¹⁶, worin R¹⁵ und R¹⁶ unabhängig voneinander für Wasserstoffatom oder -(C₁-C₄)-Alkyl stehen,
8. -C(O)-R¹⁵, worin R¹⁵ für Wasserstoffatom oder -(C₁-C₄)-Alkyl steht, oder
9. -S(O)ₓ-R¹⁵, worin x die ganze Zahl Null, 1 oder 2 bedeutet und R¹⁵ Wasserstoffatom oder -(C₁-C₄)-Alkyl bedeutet, stehen,
R22 für
1. einen Heteroarylrest aus der Gruppe 3-Hydroxypyrro-2,4-dion, Imidazol, Imidazolidin, Imidazolin, Indazol, Isothiazol, Isothiazolidin, Isoxazol, 2-Isoxazolidin, Isoxazolidin, Isoxazolon, Morpholin, Oxazol, 1,3,4-Oxadiazol, Oxadiazolidindion, Oxadiazolon, 1,2,3,5-Oxathiadiazol-2-oxid, 5-Oxo-4,5-dihydro-[1,3,4]oxadiazol, 5-Oxo-1,2,4-thiadiazol, Piperazin, Pyrazin, Pyrazol, Pyrazolin, Pyrazolidin, Pyridazin, Pyrimidin, Tetrazol, Thiadiazol, Thiazol, Thiomorpholin, Triazol oder Triazolon, steht, und
der Heteroarylrest unsubstituiert ist oder ein-, zwei- oder dreifach unabhängig voneinander substituiert ist durch
1.1 -C(O)-R¹⁵, worin R¹⁵ für Wasserstoffatom oder
-(C₁-C₄)-Alkyl steht,
1.2 -(C₁-C₄)-Alkyl,
1.3 -O-R¹⁵, worin R¹⁵ für Wasserstoffatom oder
-(C₁-C₄)-Alkyl steht,
1.4 -N(R¹⁵)-R¹⁶, worin R¹⁵ und R¹⁶ unabhängig
voneinander für Wasserstoffatom oder -(C₁-C₄)-Alkyl stehen,
1.5 Halogen oder
1.6 Keto-Rest,
2. -C(O)-R¹⁵, worin R¹⁵ für Wasserstoffatom oder -(C₁-C₄)-Alkyl steht,
3. -C(O)-OR¹⁵, worin R¹⁵ für Wasserstoffatom oder -(C₁-C₄)-Alkyl steht, oder
4. -C(O)-N(R¹⁷)-R¹⁸, worin R¹⁷ und R¹⁸ unabhängig voneinander für Wasserstoffatom, -(C₁-C₄)-Alkyl-OH, -O-(C₁-C₄)-Alkyl oder -(C₁-C₄)-Alkyl stehen,
R23 für Wasserstoffatom oder -(C₁-C₄)-Alkyl steht,
R24 für
1. einen Heteroarylrest aus der Gruppe Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolon, Oxadiazolidindion, Triazol, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, β-Carbolin und benz-anellierte, cyclopenta-, oder cyclohexa- Derivate dieser Heteroarylreste,
wobei der Heteroarylrest unsubstituiert ist oder ein-, zwei- oder dreifach unabhängig voneinander substituiert ist durch -(C₁-C₅)-Alkyl,
-(C₁-C₅)-Alkoxy, Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. einen Arylrest aus der Gruppe Phenyl, Naphthyl, 1-Naphthyl, 2-Naphthyl, Biphenylyl, 2-Biphenylyl, 3-Biphenylyl und 4-Biphenylyl, Anthryl oder Fluorenyl steht, und
der Arylrest unsubstituiert ist oder ein-, zwei- oder dreifach unabhängig voneinander substituiert ist durch -(C₁-C₅)-Alkyl, -(C₁-C₅)-Alkoxy,
Halogen, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl.

2. Verwendung der Verbindung der Formel la gemäß Anspruch 1, wobei E und M gleich oder verschieden sind und unabhängig voneinander für N-Atom oder CH stehen,
R21 und R31 gleich oder verschieden sind und unabhängig voneinander wie in Anspruch 1 unter 1. bis 9. definiert sind,
R22 für
1. einen Heteroarylrest aus der Gruppe Imidazol, Isothiazol, Isoxazol, 2-Isoxazolidin, Isoxazolidin, Isoxazolon, 1,3,4-Oxadiazol, Oxadiazolidindion, 1,2,3,5- Oxadiazolon, Oxazol, 5-Oxo-4,5-dihydro-[1,3,4]oxadiazol, Tetrazol, Thiadiazol, Thiazol, Triazol oder Triazolon steht, und der Heteroarylrest unsubstituiert ist oder ein-, zwei- oder dreifach unabhängig voneinander substituiert ist durch
1.1 Keto-Rest,
1.2 Halogen oder
1.3 -(C₁-C₂)-Alkyl, oder
2. -C(O)-N(R¹⁷)-R¹⁸, worin R¹⁷ und R¹⁸ unabhängig voneinander für Wasserstoffatom, -(C₁-C₂)-Alkyl-OH, -O-(C₁-C₂)-Alkyl oder -(C₁-C₄)-Alkyl stehen,
R23 für Wasserstoffatom, Methyl oder Ethyl steht,
R24 für
1. einen Heteroarylrest aus der Gruppe der ungesättigten, teilweise gesättigten oder vollständig gesättigte Ringe steht, die sich von Pyridin, Pyrazin, Pyrimidin, Pyridazin, Pyrrol, Furan, Thiophen, Imidazol, Pyrazol, Oxazol, Isoxazol, Thiazol, Triazol oder Isothiazol ableiten,
wobei der Heteroarylrest unsubstituiert ist oder ein-, zwei- oder dreifach unabhängig voneinander substituiert ist durch -(C₁-C₄)-Alkyl, -(C₁-C₄)-Alkoxy, F, Cl, J, Br, Nitro, Amino, Trifluormethyl, Hydroxyl, Hydroxy-(C₁-C₄)-alkyl, Methylendioxy, Ethylendioxy, Formyl, Acetyl, Cyano, Hydroxycarbonyl, Aminocarbonyl oder -(C₁-C₄)-Alkoxycarbonyl, oder
2. Phenyl steht, und Phenyl unsubstituiert ist oder ein-, zwei- oder dreifach unabhängig voneinander substituiert ist durch F, Cl, J, Br, CF₃, -OH, -(C₁-C₄)-Alkyl oder -(C₁-C₄)-Alkoxy.

3. Verwendung der Verbindung der Formel la gemäß Anspruch 1, wobei die Verbindung 2-(2-Methylamino-pyrimidin-4-yl)-1 H-indol-5-carbonsäure [(S)-2-diphenylamino-1-(5-oxo- 4,5-dihydro-[1,3,4]oxadiazol-2-yl)-ethyl]-amid oder 2-(2-Methylamino-pyrimidin-4-yl)-1 H-benzoimidazol-5-carbonsäure ((S)1-carbamoyl-2- diphenylamin-ethyl)-amid eingesetzt wird.

4. Verwendung der Verbindungen der Formel la gemäß einem oder mehreren der Ansprüche 1 bis 3 zur Herstellung von Arzneimitteln zur Prophylaxe und Therapie von akuten Schmerzen oder chronischen Schmerzen.

5. Verwendung gemäß Anspruch 4, **dadurch gekennzeichnet, dass** es sich um chronische Schmerzen aus der Gruppe chronische muskuloskeletären Erkrankungen wie Rückenschmerzen, Schmerzen bei Regelblutungen, Schmerzen bei Osteoarthrose oder Rheumatoider Arthritis, Schmerzen bei Darmentzündung, Schmerzen bei Herzmuskelentzündung, Schmerzen bei Multipler Sklerose, Schmerzen bei Neuritis, Schmerzen bei Karzinomen und Sarkomen, Schmerzen bei AIDS, Schmerzen bei Chemotherapie, Amputationsschmerz, Trigeminus-Neuralgie, Kopfschmerzen, beispielsweise Migränekopfschmez oder Neuropathische Schmerzen wie postherpetische Zostemeuralgie handelt.

6. Verwendung gemäß Anspruch 4, **dadurch gekennzeichnet, dass** es sich um akute Schmerzen aus der Gruppe Schmerzen nach Verletzungen, Postoperativer Schmerzen, Schmerzen bei akutem Gichtanfall oder akute Schmerzen nach kieferchirurgischen Eingriffen handelt.

## Claims

1. The use of the compound of the formula Ia, 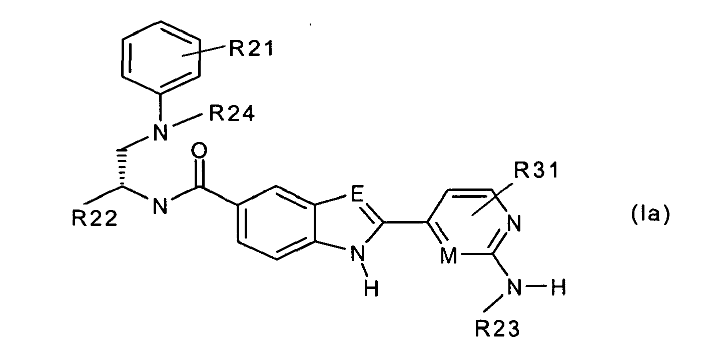 and/or a stereoisomeric form of the compound of the formula Ia and/or a physiologically tolerated salt of the compound of the formula Ia for producing pharmaceuticals for treating pain, where E and M are identical or different and are, independently of each other, N atom or CH, R21 and R31 are identical or different and are, independently of each other,
1. hydrogen atom,
2. halogen,
3. -(C₁-C₄)-alkyl,
4. -CN,
5. -CF₃,
6. -OR¹⁵, in which R¹⁵ is hydrogen atom or -(C₁-C₄)-alkyl,
7_{.} -N(R¹⁵)-R¹⁶, in which R¹⁵ and R¹⁶ are, independently of each other, hydrogen atom or -(C₁-C₄)-alkyl,
8. -C(O)-R¹⁵, in which R¹⁵ is hydrogen atom or -(C₁-C₄)-alkyl, or
9. -S(O)ₓ-R¹⁵, in which x is the integer zero, 1 or 2, and R¹⁵ is hydrogen atom or -(C₁-C₄)-alkyl,
R22 is 1. a heteroaryl radical from the group 3-hydroxypyrro-2,4-dione, imidazole, imidazolidine, imidazoline, indazole, isothiazole, isothiazolidine, isoxazole, 2-isoxazolidine, isoxazolidine, isoxazolone, morpholine, oxazole, 1,3,4-oxadiazole, oxadiazolidinedione, oxadiazolone, 1,2,3,5-oxathiadiazole-2-oxide, 5-oxo-4,5-dihydro-[1,3,4]oxadiazole, 5-oxo-1,2,4-thiadiazole, piperazine, pyrazine, pyrazole, pyrazoline, pyrazolidine, pyridazine, pyrimidine, tetrazole, thiadiazole, thiazole, thiomorpholine, triazole or triazolone, and the heteroaryl radical is unsubstituted or substituted once, twice or three times, independently of each other, by
1.1 -C(O)-R¹⁵, in which R¹⁵ is hydrogen atom or -(C₁-C₄)-alkyl,
1.2 - (C₁-C₄) -alkyl,
1.3 -O-R¹⁵, in which R¹⁵ is hydrogen atom or -(C₁-C₄)-alkyl,
1.4 -N(R¹⁵)-R¹⁶, in which R¹⁵ and R¹⁶ are, independently of each other, hydrogen atom or - (C₁-C₄) -alkyl,
1.5 halogen, or
1.6 keto radical,
2. -C(O)-R¹⁵, in which R¹⁵ is hydrogen atom or -(C₁-C₄)-alkyl,
3. -C(O)-OR¹⁵, in which R¹⁵ is hydrogen atom or -(C₁-C₄)-alkyl, or
4. -C(O)-N(R¹⁷)-R¹⁸, in which R¹⁷ and R¹⁸ are, independently of each other, hydrogen atom, -(C₁-C₄)-alkyl-OH, -O- (C₁-C₄) -alkyl or -(C₁-C₄)-alkyl,
R23 is hydrogen atom or -(C₁-C₄)-alkyl,
R24 is
1. a heteroaryl radical from the group pyrrole, furan, thiophene, imidazole, pyrazole, oxazole, isoxazole, thiazole, isothiazole, tetrazole, 1,2,3,5-oxathiadiazole-2-oxide, triazolones, oxadiazolones, isoxazolones, oxadiazolidine-dione, triazole, 3-hydroxypyrro-2,4-dione, 5-oxo-1,2,4-thiadiazole, pyridine, pyrazine, pyrimidine, indole, isoindole, indazole, phthalazine, quinoline, isoquinoline, quinoxaline, quinazoline, cinnoline, β-carboline and benzo fused cyclopenta derivatives or cyclohexa derivatives of these heteroaryl radicals,
where the heteroaryl radical is unsubstituted or substituted, once, twice or three times, independently of each other, by -(C₁-C₅)-alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl, or
2. an aryl radical from the group phenyl, naphthyl, 1-naphthyl, 2-naphthyl, biphenylyl, 2-biphenylyl, 3-biphenylyl and 4-biphenylyl, anthryl or fluorenyl, and
the aryl radical is unsubstituted or is substituted, once, twice or three times, independently of each other, by -(C₁-C₅)-alkyl, -(C₁-C₅)-alkoxy, halogen, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl.

2. The use of the compound of the formula Ia as claimed in claim 1, wherein
E and M are identical or different and are, independently of each other, N atom or CH,
R21 and R31 are identical or different and are, independently of each other, as defined in claim 1 under 1. to 9.,
R22 is
1. a heteroaryl radical from the group imidazole, isothiazole, isoxazole, 2-isoxazolidine, isoxazolidine, isoxazolone, 1,3,4-oxadiazole, oxadiazolidinedione, 1,2,3,5-oxadiazolone, oxazole, 5-oxo-4,5-dihydro[1,3,4]oxadiazole, tetrazole, thiadiazole, thiazole, triazole or triazolone, and the heteroaryl radical is unsubstituted or is substituted once, twice or three times, independently of each other, by
1.1 keto radical,
1.2 halogen or
1.3 -(C₁-C₂)-alkyl, or
2. -C(O)-N(R¹⁷)-R¹⁸, in which R¹⁷ and R¹⁸ are, independently of each other, hydrogen atom, -(C₁-C₂)-alkyl-OH, -O-(C₁-C₂)-alkyl or -(C₁-C₄)-alkyl,
R23 is hydrogen atom, methyl or ethyl,
R24 is
1. a heteroaryl radical from the group of the unsaturated, partially saturated or completely saturated rings which are derived from pyridine, pyrazine, pyrimidine, pyridazine, pyrrole, furan, thiophene, imidazole, pyrazole, oxazole, isoxazole, thiazole, triazole or isothiazole,
where the heteroaryl radical is unsubstituted or is substituted, once, twice or three times, independently of each other, by -(C₁-C₄)-alkyl, -(C₁-C₄)-alkoxy, F, Cl, I, Br, nitro, amino, trifluoromethyl, hydroxyl, hydroxy-(C₁-C₄)-alkyl, methylenedioxy, ethylenedioxy, formyl, acetyl, cyano, hydroxycarbonyl, aminocarbonyl or -(C₁-C₄)-alkoxycarbonyl, or
2. phenyl and phenyl is unsubstituted or is substituted once, twice or three times, independently of each other, by F, Cl, I, Br, CF₃, -OH, -(C₁-C₄)-alkyl or -(C₁-C₄)-alkoxy.

3. The use of the compound of the formula Ia as claimed in claim 1, wherein the compound N-[(S)-2-diphenylamino-1-(5-oxo-4,5-dihydro[1,3,4]oxadiazol-2-yl)ethyl]-2-(2-methylaminopyrimidin-4-yl)-1H-indole-5-carboxamide or N-((S)-1-carbamoyl-2-diphenylaminoethyl)-2-(2-methylaminopyrimidin-4-yl)-1H-benzimidazole-5-carboxamide is employed.

4. The use of the compound of the formula Ia as claimed in one or more of claims 1 to 3 for producing pharmaceuticals for the prophylaxis and therapy of acute pains or chronic pains.

5. The use as claimed in claim 4, wherein the chronic pains are chronic pains from the group chronic musculoskeletal diseases, such as back pains, pains associated with menstruation, pains associated with osteoarthritis or rheumatoid arthritis, pains associated with intestinal inflammation, pains associated with cardiac muscle inflammation, pains associated with multiple sclerosis, pains associated with neuritis, pains associated with carcinomas and sarcomas, pains associated with AIDS, pains associated with chemotherapy, amputation pain, trigeminus neuralgia, headaches, for example migraine cephalalgia, or neuropathic pains, such as post-herpes zoster neuralgia.

6. The use as claimed in claim 4, wherein the acute pains are acute pains from the group pains following injuries, post-operative pains, pains associated with an acute attack of gout, or acute pains following jaw-bone surgical interventions.

## Revendications

1. Utilisation du composé de formule Ia, 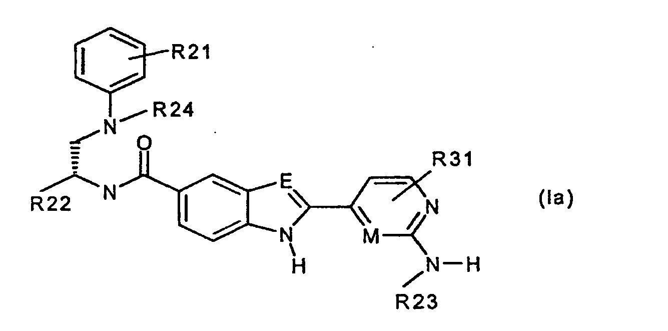 et/ou d'une forme stéréoisomérique du composé de formule Ia et/ou d'un sel physiologiquement acceptable du composé de formule Ia pour produire des préparations pharmaceutiques destinées à traiter la douleur, où
E et M sont identiques ou différents et sont, indépendamment l'un de l'autre, un atome d'azote ou CH,
R²¹ et R³¹ sont identiques ou différents et sont, indépendamment l'un de l'autre :
1. un atome d'hydrogène,
2. un halogène,
3. un alkyle en C₁-C₄,
4. -CN,
5. -CF₃,
6. -O-R¹⁵, dans lequel R¹⁵ est un atome d'hydrogène ou un alkyle en C₁-C₄,
7. -N(R¹⁵)-R¹⁶, dans lequel R¹⁵ et R¹⁶ sont, indépendamment l'un de l'autre, un atome d'hydrogène ou un alkyle en C₁-C₄,
8. -C(O)-R¹⁵, dans lequel R¹⁵ est un atome d'hydrogène ou un alkyle en C₁-C₄, ou
9. -S(O)ₓ-R¹⁵, dans lequel x est le nombre entier zéro, 1 ou 2, et R¹⁵ est un atome d'hydrogène ou un alkyle en C₁-C₄,
R²² est :
1. un radical hétéroaryle issu du groupe consistant en la 3-hydroxypyrro-2,4-dione, l'imidazole, l'imidazolidine, l'imidazoline, l'indazole, l'isothiazole, l'isothiazolidine, l'isoxazole, la 2-isoxazolidine, l'isoxazolidine, l'isoxazolone, la morpholine, l'oxazole, le 1,3,4-oxadiazole, l'oxadiazolidinedione, l'oxadiazolone, le 2-oxyde de 1,2,3,5-oxathiadiazole, le 5-oxo-4,5-dihydro-[1,3,4]oxadiazole, le 5-oxo-1,2,4-thiadiazole, la pipérazine, la pyrazine, le pyrazole, la pyrazoline, la pyrazolidine, la pyridazine, la pyrimidine, le tétrazole, le thiadiazole, le thiazole, la thiomorpholine, le triazole ou la triazolone, et
le radical hétéroaryle est non substitué ou substitué une fois, deux fois ou trois fois, indépendamment des autres, par :
1.1 -C(O)-R¹⁵, dans lequel R¹⁵ est un atome d'hydrogène ou un alkyle en C₁-C₄,
1.2 un alkyle en C₁-C₄,
1.3 -O-R¹⁵, dans lequel R¹⁵ est un atome d'hydrogène ou un alkyle en C₁-C₄,
2. 3.
4. 1.4 -N(R¹⁵)-R¹⁶, dans lequel R¹⁵ et R¹⁶ sont, indépendamment l'un de l'autre, un atome d'hydrogène ou un alkyle en C₁-C₄,
1.5 un halogène, ou
1.6 un radical cétonique,
-C (O) -R¹⁵, dans lequel R¹⁵ est un atome d'hydrogène ou un alkyle en C₁-C₄,
-C(O)-OR¹⁵, dans lequel R¹⁵ est un atome d'hydrogène ou un alkyle en C₁-C₄, ou
-C(O)-N(R¹⁷)-R¹⁸, dans lequel R¹⁷ et R¹⁸ sont, indépendamment l'un de l'autre, un atome d'hydrogène, un -(alkyle en C₁-C₄)-OH, un -O-(alkyle en C₁-C₄) ou un alkyle en C₁-C₄,
R²³ est un atome d'hydrogène ou un alkyle en C₁-C₄,
R²⁴ est :
1. un radical hétéroaryle issu du groupe consistant en le pyrrole, le furane, le thiophène, l'imidazole, le pyrazole, l'oxazole, l'isoxazole, le thiazole, l'isothiazole, le tétrazole, le 2-oxyde de 1,2,3,5-oxathiadiazole, les triazolones, les oxadiazolones, les isoxazolones, l'oxadiazolidinedione, le triazole, la 3-hydroxypyrro-2,4-dione, le 5-oxo-1,2,4-thiadiazole, la pyridine, la pyrazine, la pyrimidine, l'indole, l'isoindole, l'indazole, la phtalazine, la quinoline, l'isoquinoline, la quinoxaline, la quinazoline, la cinnoline, la β-carboline et les dérivés fusionnés avec un benzo, cyclopenta ou cyclohexa de ces radicaux hétéroaryle,
où le radical hétéroaryle est non substitué ou substitué, une fois, deux fois ou trois fois, indépendamment des autres, par un alkyle en C₁-C₅, un alcoxy en C₁-C₅, un halogène, un nitro, un amino, un trifluorométhyle, un hydroxyle, un
hydroxy-(alkyle en C₁-C₄), un méthylènedioxy, un éthylènedioxy, un formyle, un acétyle, un cyano, un hydroxycarbonyle, un aminocarbonyle ou un alcoxycarbonyle en C₁-C₄, ou
2. un radical aryle issu du groupe consistant en le phényle, le naphtyle, le 1-naphtyle, le 2-naphtyle, le biphénylyle, le 2-biphénylyle, le 3-biphénylyle et le 4-biphénylyle, l'anthryle ou le fluorényle, et
le radical aryle est non substitué ou est substitué, une fois, deux fois ou trois fois, indépendamment des autres, par un alkyle en C₁-C₅, un alcoxy en C₁-C₅, un halogène, un nitro, un amino, un trifluorométhyle, un hydroxyle, un hydroxy-(alkyle en C₁-C₄), un méthylènedioxy, un éthylènedioxy, un formyle, un acétyle, un cyano, un hydroxycarbonyle, un aminocarbonyle ou un alcoxycarbonyle en C₁-C₄.

2. Utilisation du composé de formule Ia selon la revendication 1, dans laquelle :
E et M sont identiques ou différents et sont, indépendamment l'un de l'autre, un atome d'azote ou CH,
R²¹ et R³¹ sont identiques ou différents et sont, indépendamment l'un de l'autre, comme défini dans la revendication 1, du 1. au 9.,
R²² est :
1. un radical hétéroaryle issu du groupe consistant en l'imidazole, l'isothiazole, l'isoxazole, la 2-isoxazolidine, l'isoxazolidine, l'isoxazolone, le 1,3,4-oxadiazole, l'oxadiazolidinedione, la 1,2,3,5-oxadiazolone, l'oxazole, le 5-oxo-4,5-dihydro-[1,3,4]oxadiazole, le tétrazole, le thiadiazole, le thiazole, le triazole ou la triazolone, et le radical hétéroaryle est non substitué ou est
2. substitué une fois, deux fois ou trois fois, indépendamment des autres, par :
1.1 un radical cétonique,
1.2 un halogène ou
1.3 un alkyle en C₁-C₂, ou
-C(O)-N(R¹⁷)-R¹⁸, dans lequel R¹⁷ et R¹⁸ sont, indépendamment l'un de l'autre, un atome d'hydrogène, un -(alkyle en C₁-C₂) -OH, un -O-(alkyle en C₁-C₂) ou un alkyle en C₁-C₄,
R²³ est un atome d'hydrogène, un méthyle ou un éthyle,
R²⁴ est :
1. 2.
un radical hétéroaryle issu du groupe consistant en les cycles insaturés, partiellement saturés ou complètement saturés qui sont dérivés de la pyridine, de la pyrazine, de la pyrimidine, de la pyridazine, du pyrrole, du furane, du thiophène, de l'imidazole, du pyrazole, de l'oxazole, de l'isoxazole, du thiazole, du triazole ou de l'isothiazole,
où le radical hétéroaryle est non substitué ou est substitué, une fois, deux fois ou trois fois, indépendamment des autres, par un alkyle en C₁-C₄, un alcoxy en C₁-C₄, F, Cl, I, Br, un nitro, un amino, un trifluorométhyle, un hydroxyle, un hydroxy-(alkyle en C₁-C₄), un méthylènedioxy, un éthylènedioxy, un formyle, un acétyle, un cyano, un hydroxycarbonyle, un aminocarbonyle, ou un alcoxycarbonyle en C₁-C₄, ou
un phényle et le phényle est non substitué ou est substitué une fois, deux fois ou trois fois, indépendamment des autres, par F, Cl, I, Br, CF₃, -OH, un alkyle en C₁-C₄ ou un alcoxy en C₁-C₄.

3. Utilisation du composé de formule Ia selon la revendication 1, dans laquelle le composé N-[(S)-2-diphénylamino-1-(5-oxo-4,5-dihydro[1,3,4]oxadiazol-2-yl)éthyl]-2-(2-méthylaminopyrimidin-4-yl)-1H-indole-5-carboxamide ou N-((S)-1-carbamoyl-2-diphénylaminoéthyl)-2-(2-méthylaminopyrimidin-4-yl)-1H-benzimidazole-5-carboxamide est utilisé.

4. Utilisation du composé de formule Ia selon l'une ou plusieurs des revendications 1 à 3 pour produire des préparations pharmaceutiques destinées à la prophylaxie et à la thérapie des douleurs aiguës ou des douleurs chroniques.

5. Utilisation selon la revendication 4, dans laquelle les douleurs chroniques sont des douleurs chroniques du groupe des maladies musculosquelettiques chroniques, telles que les douleurs dorsales, des douleurs liées à la menstruation, les douleurs liées à l'ostéoarthrite ou l'arthrite rhumatoïde, les douleurs liées à l'inflammation intestinale, les douleurs liées à l'inflammation du muscle cardiaque, les douleurs liées à la sclérose en plaques, les douleurs liées à la névrite, les douleurs liées aux carcinomes et aux sarcomes, les douleurs liées au SIDA, les douleurs liées à la chimiothérapie, la douleur due à une amputation, la névralgie trigéminée, les maux de tête, par exemple la migraine céphalée, ou les douleurs neuropathiques, telles que la névralgie post-zostérienne.

6. Utilisation selon la revendication 4, dans laquelle les douleurs aiguës sont des douleurs aiguës du groupe des douleurs survenant à la suite de blessures, des douleurs postopératoires, des douleurs liées à une attaque aiguë de goutte, ou des douleurs aiguës survenant à la suite d'interventions chirurgicales de la mâchoire.
